(19) Europäisches Patentamt
European Patent Office
Office européen des brevets

(11) **EP 4 530 299 A1**

(12) **EUROPEAN PATENT APPLICATION**
published in accordance with Art. 153(4) EPC

(43) Date of publication:
02.04.2025 Bulletin 2025/14

(21) Application number: 23810691.8

(22) Date of filing: 14.04.2023

(51) International Patent Classification (IPC):
*C07K 16/46* (2006.01)   *C07K 16/28* (2006.01)
*C12N 15/13* (2006.01)   *A61K 39/395* (2006.01)
*A61P 11/06* (2006.01)

(52) Cooperative Patent Classification (CPC):
A61K 39/395; A61P 11/06; C07K 16/00;
C07K 16/28; C07K 16/46

(86) International application number:
PCT/CN2023/088385

(87) International publication number:
WO 2023/226617 (30.11.2023 Gazette 2023/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 23.05.2022 CN 202210561675

(71) Applicant: Regenecore Biotech Co., Ltd
Nanjing, Jiangsu 210000 (CN)

(72) Inventors:
• SU, Zhipeng
  Nanjing, Jiangsu 210000 (CN)
• WANG, Yang
  Nanjing, Jiangsu 210000 (CN)
• XIE, Wei
  Nanjing, Jiangsu 210000 (CN)

(74) Representative: Murgitroyd & Company
165-169 Scotland Street
Glasgow G5 8PL (GB)

Remarks:
The complete document including Reference
Table(s) and the Sequence Listing(s) can be
downloaded from the EPO website

(54) **STABLE ANTIBODY PREPARATION**

(57) The present invention belongs to the field of immunology, and relates to a stable antibody preparation. The stable antibody preparation comprises a therapeutically effective amount of an antibody molecule, a tonicity agent, a buffer agent, a filler and a surfactant, and the preparation has a pH of 4.5-5.5. The antibody molecule can simultaneously specifically bind to IL-5 and IL-4 Ra, and comprises at least two immunoglobulin variable domains, which are divided into a first immunoglobulin variable domain capable of specifically binding to IL-4 Ra molecule and a second immunoglobulin variable domain n capable of specifically binding to IL-5. The antibody in the antibody preparation substantially retains the physical and chemical stability, and integrity thereof during storage.

FIG. 6

**Description**

TECHNICAL FIELD

The present disclosure relates to the field of medicine. More specifically, the present disclosure relates to a stable antibody formulation.

BACKGROUND

With the advancement of biotechnology, it is possible to produce a variety of proteins for pharmaceutical applications by using recombinant DNA technology. Because proteins tend to be larger and more complex than traditional organic or inorganic drugs, special problems are found in the formulation of these proteins.

Liquid antibody products have a short shelf life and the antibody may biologically inactivate due to chemical and physical instability during storage. Chemical instability may result from deamidation, racemization, hydrolysis, oxidation, beta-elimination, or disulfide bond exchange, and physical instability may result from antibody denaturation, aggregation, precipitation, or adsorption. Among them, aggregation, deamidation and oxidation are known to be the most common causes of antibody degradation (Wang et al., 1988, J. of parenteral Science & Technology 42(Suppl): S4-S26; Cleland et al., 1993, Critical Reviews in Therapeutic Drug Carrier Systems 10(4): 307-377). Therefore, there is a need for stable liquid formulations of antibodies, and in particular, for stable liquid formulations of high-concentration antibodies.

SUMMARY

It is an object of the present disclosure to provide an antibody formulation which is stable upon storage and delivery. According to the present disclosure, a stable formulation is a formulation wherein the antibody therein essentially retains its physical and chemical stability and integrity upon storage. It is a further object to provide a stable liquid antibody formulation which is suitable for administration such as subcutaneous administration and intravenous drip infusion.

In general, it is preferred to use small volumes of pharmaceutical formulation for subcutaneous injection. In the case of formulations including antibodies, e.g. in high dose antibody therapies, the subcutaneous administration requires high-concentration antibody formulations. Because of the required high antibody concentrations, the formulations including antibodies pose challenges relating to the physical and chemical stability of the antibody, formation of aggregates and difficulty with manufacture, storage, and delivery of the antibody formulation.

The formation of by-products and degradation products and biologically inactivation are generally main factors that determines shelf life. The formulation of the present disclosure achieves these desired stability levels.

Apart from sufficient physical and chemical stability, the formulation should be of acceptable pH value and osmolality for application. It is also known that a high concentration of antibodies would increase the viscosity of the formulation and also the aggregation. Suitable pharmaceutical formulations according to the present disclosure have a desired suitable viscosity.

In accordance with the present disclosure, it has been found that particularly stable antibody formulations are available and have advantageous properties in terms of preserving antibody activity during long-term storage, preventing aggregation and having a suitable viscosity despite high antibody concentrations. The present disclosure provides in its broadest aspect a pharmaceutical formulation (formulation of the present disclosure) including an antibody as an active ingredient, a buffer system, a stabilizer and a surfactant. The formulation of the present disclosure is liquid (e.g., aqueous solution) but is also suitable to be lyophilized and subsequently formed into a liquid formulation with a lower, same or higher antibody concentration.

1. OUTLINE

1.1 A first aspect of the present disclosure provides the following stable formulations.

> (1) A formulation including at least one therapeutically effective amount of an antibody molecule, at least one tonicity agent, at least one buffer agent, and at least one surfactant, wherein the composition (i.e., the formulation) has a pH of 4.5 to 5.5.
> (2) A formulation including at least one therapeutically effective amount of an antibody molecule, at least one tonicity agent, at least one buffer agent, and at least one surfactant, wherein the composition has a pH of 4.7 to 5.3.
> (3) A formulation consisting of substantially consisting of at least one therapeutically effective amount of an antibody molecule, at least one tonicity agent, at least one buffer agent, and at least one surfactant, wherein the composition has a pH of 5.0 to 5.2.

The antibody molecules in the compositions (1) to (3) are capable of specifically binding to both IL-5 and IL-4Rα, and include at least two immunoglobulin variable domains including a first immunoglobulin variable domain capable of specifically binding to IL-4Rα molecule and a second immunoglobulin variable domain capable of specifically binding to IL-5.

"Treatment" refers to both therapeutic treatment and prophylactic treatment. Those in need of treatment include those have been suffering from the disorder as well as those in which the disorder is to be prevented. "Mammal" for purposes of treatment refers to any animal classified as a mammal, including but not limited to humans, domestic and farm animals, and zoo, sport, or pet animals, such as dogs, horses, cats, and cattle.

In a pharmacological sense, in the context of the present disclosure, a "therapeutically effective amount" of an antibody refers to an amount at which an antibody has a therapeutic or prophylactic effect on the disorder that can be effectively treated with the antibody. A "disorder" is any condition that would benefit from treatment with the antibody. This includes chronic and acute disorders or diseases including those pathological conditions which predispose the mammal to the disorder in question. In a preferred embodiment, "disorder" is a disease involving expressing IL-4R and/or IL-5.

The formulation according to the present disclosure provides the advantage that it stably supports high concentrations of bioactive antibody in solution and is suitable for parenteral administration, including intravenous, intramuscular, or subcutaneous injection.

According to a preferred embodiment of the present disclosure, the pH can be in a range of 4.5 to 5.5, preferably between about pH 4.5 and any of about pH 4.6, 4.7, 4.8, 4.9, 5.0, 5.1, 5.2, 5.3, 5.4, or 5.5.

Preferably, the pH is selected from pH values between pH 4.6 and any of about pH 4.7, 4.8, 4.9, 5.0, 5.1, 5.2, 5.3, 5.4, or 5.5; more preferably, the pH is selected from pH values between about pH 4.7 and any of about pH 4.8, 4.9, 5.0, 5.1, 5.2, 5.3, 5.4, or 5.5; more preferably, the pH is selected from pH values between about pH 4.8 and any of about pH 4.9, 5.0, 5.1, 5.2, 5.3, 5.4, or 5.5; more preferably, the pH is selected from pH values between about pH 4.9 and any of pH 5.0, 5.1, 5.2, 5.3, 5.4, or 5.5; more preferably, the pH is selected from pH values between pH 5.0 and any of pH 5.1, 5.2, 5.3, 5.4, or 5.5; more preferably, the pH is selected from pH values between pH 5.1 and any of pH 5.2, 5.3, 5.4, or 5.5. In a preferred embodiment, the pH can be in a range of about pH 5.2 to any of pH 5.3, 5.4, or 5.5, or the pH can be in a range of about pH 5.3 to pH 5.4, or pH 5.5, or in a range of pH5.4 to 5.5.

More preferably, the pH can be selected from any pH values of about 4.5, 4.6, 4.7, 4.8, 4.9, 5.0, 5.1, 5.2, 5.3, 5.4, or 5.5, most preferably the pH is pH 5.0 ± 0.2. The pH values in these ranges provides the formulation with enhanced protection from antibody aggregation and fragmentation and is close to physiological pH (about pH 7.2 to 7.4) for reduced risk of pain or anaphylactoid side effects upon injection.

According to a further preferred embodiment of the present disclosure, the tonicity agent preferably includes a polyol, a saccharide, a carbohydrate, a salt, such as sodium chloride, or mixtures thereof. Preferably the polyol has a molecular weight that is less than about 600 kD (e.g., in a range from about 120 to about 400 kD), preferably selected from mannitol, trehalose, sorbitol, erythritol, isomalt, lactitol, maltitol, xylitol, glycerol, lactitol, propylene glycol, polyethylene glycol, inositol, or mixtures thereof. Preferably the saccharide or carbohydrate is selected from the group consisting of monosaccharides, disaccharides and polysaccharides and mixtures thereof. Preferably the saccharide or carbohydrate is selected from the group consisting of fructose, glucose, mannose, sucrose, sorbose, xylose, lactose, maltose, sucrose, dextran, pullulan, dextrin, cyclodextrins, soluble starch, hydroxyethyl starch, water- soluble glucans, and mixtures thereof. Preferably the tonicity agent includes a saccharide selected from the group consisting of reducing sugar, non-reducing sugar and mixtures thereof. Further preferably the tonicity agent includes a saccharide which is a non-reducing sugar, preferably selected from the group consisting of sucrose, trehalose, and mixtures thereof. Most preferably the tonicity agent includes sucrose.

A concentration of the tonicity agent ranges from about 1 mg/ml to about 300 mg/ml, from about 1 mg/ml to about 200 mg/ml, or from about 1 mg/ml to about 100 mg/ml. Preferably the concentration of the tonicity agent in the liquid composition is about 60 mg/ml, about 65 mg/ml, about 70 mg/ml, about 75 mg/ml, about 80 mg/ml, about 81 mg/ml, about 82 mg/ml, about 83 mg/ml, about 84 mg/ml, about 85 mg/ml, about 86 mg/ml, about 87 mg/ml, about 88 mg/ml, about 89 mg/ml, about 90 mg/ml, about 91 mg/ml, about 92 mg/ml, about 93 mg/ml, about 94 mg/ml, about 95 mg/ml, about 96 mg/ml, about 97 mg/ml, about 98 mg/ml, about 99 mg/ml, about 100 mg/ml, about 105 mg/ml, about 110 mg/ml, about 120 mg/ml, or about 130 mg/ml.

According to a preferred embodiment of the present disclosure, the buffer agent can be selected from the group consisting of acetate, succinate, gluconate, citrate, histidine, acetic acid, phosphate, phosphoric acid, ascorbate, tartartic acid, maleic acid, glycine, lactate, lactic acid, ascorbic acid, imidazole, bicarbonate and carbonic acid, succinic acid, sodium benzoate, benzoic acid, gluconate, edetate, acetate, malate, imidazole, tris, phosphate, and mixtures thereof.

According to the present disclosure, the buffer agent, particularly acetate buffer as a preferred buffer agent, provides the liquid composition with a pH close to physiological pH for reduced risk of pain or anaphylactoid side effects upon injection and also provides enhanced antibody stability and resistance to aggregation, oxidation and fragmentation.

A concentration of the buffer agent can range from about 0.1 millimolar (mM) to about 100 mM. Preferably, the concentration of the buffer agent is from about 0.5 mM to about 50 mM, further preferably about 1 mM to about 30 mM, more preferably about 1 mM to about 20 mM, or more preferably about 10 mM to about 20 mM. Preferably, the concentration of the buffer agent is about 1 mM, about 2 mM, about 3 mM, about 4 mM, about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 10 mM, about 18 mM, about 19 mM, or about 20 mM.

As used herein, the term "surfactant" refers to organic substances having amphipathic structures; i.e., they are composed of groups of opposing solubility tendencies, typically an oil-soluble hydrocarbon chain and a water-soluble ionic group. Surfactants can be classified, depending on the charge of the surface-active moiety, into anionic surfactant, cationic surfactant and dispersing agents for various pharmaceutical compositions and preparations of biological materials.

According to a further preferred embodiment of the present disclosure, the suitable surfactant for use in the disclosure includes, but is not limited to, a non-ionic surfactant, an ionic surfactant and a zwitterionic surfactant. The typical surfactant for use in the present disclosure includes, but is not limited to, sorbitan fatty acid esters (e.g. sorbitan monocaprylate, sorbitan monolaurate, sorbitan monopalmitate), sorbitan trioleate, glycerine fatty acid esters (e.g. glycerine monocaprylate, glycerine monomyristate, glycerine monostearate), polyglycerine fatty acid esters (e.g. decaglyceryl monostearate, decaglyceryl distearate, decaglyceryl monolinoleate), polyoxyethylene sorbitan fatty acid esters (e.g. polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate), polyoxyethylene sorbitol fatty acid esters (e.g. polyoxyethylene sorbitol tetrastearate, polyoxyethylene sorbitol tetraoleate), polyoxyethylene glycerine fatty acid esters (e.g. polyoxyethylene glyceryl monostearate), polyethylene glycol fatty acid esters (e.g. polyethylene glycol distearate), polyoxyethylene alkyl ethers (e.g. polyoxyethylene lauryl ether), polyoxyethylene polyoxypropylene alkyl ethers (e.g. polyoxyethylene polyoxypropylene glycol, polyoxyethylene polyoxypropylene propyl ether, polyoxyethylene polyoxypropylene cetyl ether), polyoxyethylene alkylphenyl ethers (e.g. polyoxyethylene nonylphenyl ether), polyoxyethylene hydrogenated castor oils (e.g. polyoxyethylene castor oil, polyoxyethylene hydrogenated castor oil), polyoxyethylene beeswax derivatives (e.g. polyoxyethylene sorbitol beeswax), polyoxyethylene lanolin derivatives (e.g. polyoxyethylene lanolin), and polyoxyethylene fatty acid amides (e.g. polyox-yethylene stearic acid amide); $C_{10}$-$C_{18}$ alkyl sulfates (e.g. sodium cetyl sulfate, sodium lauryl sulfate, sodium oleyl sulfate), polyoxyethylene $C_{10}$-$C_{18}$ alkyl ether sulfate with an average of 2 to 4 moles of ethylene oxide units added (e.g. sodium polyoxyethylene lauryl sulfate), and $C_1$-$C_{18}$ alkyl sulfosuccinate (e.g. sodium lauryl sulfosuccinate); and natural surfactant such as lecithin, glycerophospholipid, sphingophospholipids (e.g. sphingomyelin), and sucrose esters of $C_{12}$-$C_{18}$ fatty acids. A composition may include one or more of these surfactants. A preferred surfactant is polyoxyethylene sorbitan fatty acid esters e.g. polysorbate 20, 40, 60 or 80. Polysorbate 80 (Tween 80) is particularly useful. According to the present disclosure, the surfactant, particularly polysorbate 80, provides the liquid composition with enhanced antibody stability and resistance to aggregation and fragmentation.

A concentration of the surfactant generally ranges from about 0.01 mg/ml to about 10 mg/ml, from about 0.01 mg/ml to about 5.0 mg/ml, from about 0.01 mg/ml to about 2.0 mg/ml, from about 0.01 mg/ml to about 1.5 mg/ml, from about 0.01 mg/ml to about 01.0 mg/ml, from about 0.01 mg/ml to about 0.5 mg/ml, from about 0.01 mg/ml to about 0.4 mg/ml, from about 0.01 mg/ml to about 0.3 mg/ml, from about 0.01 mg/ml to about 0.2 mg/ml, from about 0.01 mg/ml to about 0.15 mg/ml, from about 0.01 mg/ml to about 0.1 mg/ml, or from about 0.01 mg/ml to about 0.05 mg/ml. Further preferably, the concentration of the surfactant is about 5 mg/ml, about 0.5 mg/ml, about 0.6 mg/ml, about 0.7 mg/ml, about 0.8 mg/ml, about 0.9 mg/ml, about 1 mg/ml, about 1.1 mg/ml, about 1.2 mg/ml, about 1.3 mg/ml, about 1.4 mg/ml, about 1.5 mg/ml, about 1.6 mg/ml, about 1.7 mg/ml, about 1.8 mg/ml, about 1.9 mg/ml, or about 2 mg/ml.

1.2 In some embodiments of the present disclosure, the first immunoglobulin variable domain is a first single-domain antibody molecule, wherein the first single-domain antibody molecule binds to IL-4Ra and includes three complementary determining regions (CDRs), CDR1 having an amino acid sequence as set forth in SEQ ID NO: 1, CDR2 having an amino acid sequence as set forth in SEQ ID NO: 2, and CDR3 having an amino acid sequence as set forth in SEQ ID NO: 3, or CDRs having less than 3, 2 or 1 amino acid substitution (e.g., conservative substitution) as compared to said CDRs. The second immunoglobulin variable domain is a second single-domain antibody molecule, wherein the second single-domain antibody molecule binds to IL-5 and includes three complementary determining regions (CDRs), CDR1 having an amino acid sequence as set forth in SEQ ID NO: 4, CDR2 having an amino acid sequence as set forth in SEQ ID NO: 5, and CDR3 having an amino acid sequence as set forth in SEQ ID NO: 6, or CDRs having less than 3, 2 or 1 amino acid substitution (e.g., conservative substitution) as compared to said CDRs.

1.3 In some embodiments of the present disclosure, the antibody molecule is a polypeptide fusion including no linker group between the first single-domain antibody molecule and the second single-domain antibody molecule.

1.4 In some embodiments of the present disclosure, the antibody molecule is a polypeptide fusion including a biocompatible polymer with 1-100 atoms as a linker group via which the first single-domain antibody molecule and the second single-domain antibody molecule are ligated. The linker group can be any linker group apparent to those of skill in the art. In an embodiment, the linker group includes or consists of polyglycine, polyalanine, polyserine, polylysine, polyglutamate, polyisoleucine, or polyarginine residues, or a combination thereof. For example, the polyglycine or polyserine linkers can include at least five, seven, eight, nine, ten, twelve, fifteen, twenty, thirty, thirty-five and forty glycine and serine residues. Exemplary linkers that can be used include Gly-Ser repeats, for example, (Gly)4-Ser repeats of one, two, three, four, five, six, seven or more repeats. In many embodiments, the linker has the following sequence: (Gly)4- Ser-(Gly)3-Ser or ((Gly)4-Ser)n, where n is 4, 5, or 6.

1.5 In some embodiments, the first single-domain antibody molecule and the second single-domain antibody molecule are ligated via a human IgG Fc region. The antibody molecule, which has a homodimer structure and is named 4E9V10-2B3V2, includes two identical single-chain Fc fusion proteins. Each of the single-chain Fc fusion proteins is composed of a first immunoglobulin variable domain, a human IgG Fc region, and a second immunoglobulin variable domain. Each single-chain Fc fusion protein is referred to as each single-chain for short.

A process of preparing the above antibodies includes introducing a gene sequence encoding anti-IL-4Rα single-domain antibody or a gene sequence encoding anti-IL-5 single-domain antibody into a vector (obtained referring to the method disclosed in Chinese invention patent application no. CN202010576200.7 titled with "ANTI-IL-4RA SINGLE-DOMAIN ANTIBODY AS WELL AS APPLICATION AND DRUG"), respectively, through sequence synthesis, and producing a dual chain antibody molecule through genetic recombination technology.

The first single-domain antibody molecule and the second immunoglobulin variable domain can be derived from molecules naturally devoid of light chains (e.g., VHH, nanobodies, or camelid-derived antibodies). The single-domain antibody molecule can be derived or obtained from camelids such as camel, llama, dromedary, alpaca or guanaco. In other embodiments, the SDAB molecule may include single-domain molecules including, but not limited to, other naturally-occurring single-domain molecules, such as shark single-domain polypeptides (Shark Immunoglobulin New Antigen Receptors (IgNAR)); and single-domain scaffolds (e.g., fibronectin scaffolds). Single-domain molecules may be derived from sharks.

1.6 In some embodiments of the present disclosure, the antibody molecule is a dual-chain antibody molecule composed of two identical single-chain Fc fusion proteins paired and ligated with each other. each of the single-chain Fc fusion proteins is composed of a first immunoglobulin variable domain, a human IgG Fc region, and a second immunoglobulin variable domain.

In the present application, one of the antigens that the bispecific antibody can recognize is IL-5 protein, and the other is IL-4Rα protein.

1.7 In some embodiments of the present disclosure, the human IgG Fc region is derived from human IgG1, IgG2, IgG3 or IgG4.

1.8 In some embodiments of the present disclosure, the human IgG Fc region is amino acids derived from human IgG4 region; and the Fc region has an amino acid sequence as set forth in SEQ ID NO: 13, or at positions 116 to 356 of SEQ ID NO: 9.

1.9 In some embodiments of the present disclosure, the first immunoglobulin variable domain has an amino acid sequence as set forth in SEQ ID NO: 7, and the second immunoglobulin variable domain has an amino acid sequence as set forth in SEQ ID NO: 8.

The first immunoglobulin variable domain as set forth in SEQ ID NO: 7 is a humanized single-domain antibody molecule. The second immunoglobulin variable domain as set forth in SEQ ID NO: 8 is a humanized single-domain antibody molecule. SEQ ID NO: 7 (4E9V10) is derived from humanization of the amino acid sequence of single-domain antibody 4E9 of CN202010576200.7 titled with "ANTI-IL-4RA SINGLE-DOMAIN ANTIBODY AS WELL AS APPLICATION AND DRUG". SEQ ID NO: 8 (2B3V2) is derived from humanization of the amino acid sequence of single-domain antibody 2B3 of CN202010843501.1 titled with IL-5 BINDING MOLECULES AND PREPARATION METHODS AND APPLICATIONS THEREOF. The humanization method of single-domain antibody 2B3 is described in CN202010843501.1 titled with IL-5 BINDING MOLECULES AND PREPARATION METHODS AND APPLICATIONS THEREOF.

A humanization method of the single-domain antibody 4E9 is to humanize by high-throughput screening of an antibody framework region mutation library constructed based on the results of big data analysis. The detailed steps are as follows:

(1) sequence analysis of data of human/camel antibodies: conduct amino acid preference analysis is carried out on sequences of 13,873 Nb cases (Human) downloaded in batches from the NCBI website and amino acid preference analysis is carried out on sequences of 2,000 single-domain antibodies from the applicant to obtain amino-terminal proportion data for each site in the framework region;

(2) comprehensive weighted analysis of human/camel antibodies: the sequences of the above-mentioned human/-camel antibodies according to the IMGT numbering rules are numbered, the sequences are matched one-to-one, a weighted analysis is conducted based on the weight of 90% of human antibodies and 10% of camel antibodies, the weighted proportions of amino acids at each site are calculated, the proportion are ranked from high to low, only amino acid species accounting for >10% are retained according to the final weighted result at a single site in the framework region, and the final weights of amino acids with a proportion >10% are calculated based on the standard that the overall retained proportion is 1, as the basis for the design of subsequent customized amino acid libraries;

(3) scheme design of customized amino acid library: for a single site to be mutated, the number of amino acids accounting for >10% is specified as n, and the ratio of the highest proportion value to the lowest proportion value of amino acids accounting for >10% is specified as V; and the nature of the site to be mutated are determined: if V≥3 and n≤2, the site is considered to be a "high concentration site"; otherwise, the site is considered to be a "medium-low concentration site". According to this method, the customized amino acid libraries are divided into "high/medium and low concentration libraries", both of which are used for constructing customized amino acid libraries respectively. The final weight in (2) above is the reference basis for the types and proportions of amino acid species in the libraries.

(4) High-throughput screening of customized amino acid libraries:

A humanized antibody library is constructed for non-humanized 4E9, and the constructed libraries are panned with corresponding antigens, thereby finally obtaining an antibody sequence with higher affinity and a higher degree of humanization.

Preferably, 4E9V10 has a structure of FR1-CDR1-FR2-CDR2-FR3-CDR3-FR4. FR1 has an amino acid sequence of SEQ ID NO: 14; FR2 has an amino acid sequence of SEQ ID NO: 15; FR3 has an amino acid sequence of SEQ ID NO: 16; and FR4 has an amino acid sequence of SEQ ID NO: 17.

2B3V2 has a structure of FR1-CDR1-FR2-CDR2-FR3-CDR3-FR4. FR1 has an amino acid sequence of SEQ ID NO: 18; FR2 has an amino acid sequence of SEQ ID NO: 19; FR3 has an amino acid sequence of SEQ ID NO: 20; and FR4 has an amino acid sequence of SEQ ID NO: 21.

Herein, a "humanized antibody" refers to an antibody obtained by fusing the heavy chain variable domain of a target antibody (such as an animal antibody) with the constant region of a human antibody, an antibody obtained by grafting the complementarity-determining region (CDR 1-3 sequences) of a target antibody into the variable region of a human antibody, or an antibody obtained by subjecting a target antibody to amino acid mutation according to the characteristics of the framework region (FRs 1-4) of a human antibody. The humanized antibody may be obtained through synthesis or site-directed mutagenesis.

A process of preparing the above antibodies in item 1.9 can include introducing a gene sequence (nucleotides at positions 1 to 345 of SEQ ID NO: 11 or 12) of anti-IL-4Rα humanized single-domain antibody (4E9V10) or a gene sequence (nucleotides at positions 1069 to 1434 of SEQ ID NO: 11 or 12) of anti-IL-5 humanized single-domain antibody (named 2B3V2) into a vector (obtained referring to the method disclosed in Chinese invention patent application no. CN202010576200.7 titled with "ANTI-IL-4RA SINGLE-DOMAIN ANTIBODY AS WELL AS APPLICATION AND DRUG"), respectively, through sequence synthesis, to obtain a genetically modified plasmid, and expressing a dual chain antibody from the plasmids.

1.10 In some embodiments of the present disclosure, a stable antibody formulation is provided that includes a therapeutically effective amount of an antibody molecule, a tonicity agent, a buffer agent, and a surfactant, and the formulation has a pH of 4.5 to 5.5; the antibody molecule includes a fusion protein with the amino acid sequence as set forth in SEQ ID NO: 9 or 10. Preferably, the antibody molecule is a dual chain antibody, and each single chain constituting the dual chain antibody molecule has an amino acid sequence as set forth in SEQ ID NO. 9 or SEQ ID NO. 10. The two single-chain Fc regions automatically pair up to form a dual chain antibody molecule.

In some embodiments of the present disclosure, the antibody molecule, referred to herein as "4E9V10-2B3V2", includes, or consists of, the amino acid sequence as set forth in SEQ ID NO: 9 or 10, or an amino acid sequence substantially identical thereto (e.g., an amino acid sequence at least 85%, 90%, 95% or more identical to, or having up to 20, 15, 10, 5, 4, 3, 2, 1 amino acid alterations (e.g., deletions, insertions or substitutions (e.g., conservative substitutions) relative to the amino acid sequence as set forth in SEQ ID NO: 9 or 10)).

4E9V10-2B3V2 has an amino acid sequence as set forth in SEQ ID NO: 9 or 10, and the corresponding nucleotide sequence is as set forth in SEQ ID NO: 11 or 12. 4E9V10-2B3V2 includes three segments: 4E9V10-Fc segment-2B3V2, wherein 4E9V10 is located at the amino terminus (positions 1 to 115 of SEQ ID NO: 9 or 10), Fc segment is located at positions 116 to 356 of SEQ ID NO: 9 or 10, and 2B3V2 is located at the carboxyl terminus (positions 357 to 478 of SEQ ID NO: 9 or 10).

4E9V10-2B3V2 includes 4E9V10-2B3V2-1 (i.e., having an amino acid sequence as set forth in SEQ ID NO: 9, and a nucleotide sequence as set forth in SEQ ID NO: 11) and 4E9V10-2B3V2-2 (i.e., having an amino acid sequence as set forth in SEQ ID NO: 10, and a nucleotide sequence as set forth in SEQ ID NO: 12).

2. Formula

2.1 In some embodiments of the present disclosure, the formulation buffer agent in item 1.1 is an acetic acid-sodium acetate buffer, the tonicity agent includes sucrose, and the surfactant includes polysorbate.

2.2 In some embodiments of the present disclosure, the formulation further includes a filler including glycine, and arginine hydrochloride.

2.3 Acetic acid-sodium acetate: In some embodiments of the present disclosure, the formulation (optionally) includes acetic acid-sodium acetate at a concentration of 10 to 20 mmol/L, for example, at a concentration of about 10 mmol/L to 19 mmol/L, about 10 mmol/L to 18 mmol/L, about 10 mmol/L to 17 mmol/L, about 10 mmol/L to 16 mmol/L, about 10 mmol/L to 15 mmol/L, about 10 mmol/L to 14 mmol/L, about 10 mmol/L to 13 mmol/L, about 10 mmol/L to 12 mmol/L, about 10 mmol/L to 11 mmol/L, about 10 mmol/L, about 11 mmol/L, about 12 mmol/L, about 13 mmol/L, about 14 mmol/L, 15 mmol/L, 16 mmol/L, 17 mmol/L, 18 mmol/L, 19 mmol/L, or 20 mmol/L. The overall concentration of acetic acid in the formulation and sodium acetate in the formulation is 10 to 20 mmol/L.

Preferably, a mass ratio of acetic acid to sodium acetate is (0.2 to 2) : (1 to 10). More preferably, the mass ratio of acetic acid to sodium acetate is 2 : 9.

2.4 Arginine hydrochloride: In some embodiments of the present disclosure, the formulation (optionally) includes arginine hydrochloride at a concentration of 25 to 100 mmol/L, for example, at a concentration of about 25 mmol/L to 90 mmol/L, about 25 mmol/L to 80 mmol/L, about 25 mmol/L to 70 mmol/L, about 25 mmol/L to 60 mmol/L, about 25 mmol/L to 50 mmol/L, about 25 mmol/L to 40 mmol/L, about 25 mmol/L to 30 mmol/L, about 25 mmol/L, about 30 mmol/L, about 40 mmol/L, about 50 mmol/L, about 60 mmol/L, 70 mmol/L, 80 mmol/L, 90 mmol/L, or 100 mmol/L.

2.5 Sucrose: In some embodiments of the present disclosure, the formulation (optionally) includes sucrose as a tonicity agent at a concentration of 80 to 120 mmol/L, for example, at a concentration of about 80 mmol/L to 118 mmol/L, about 80 mmol/L to 115 mmol/L, about 80 mmol/L to 110 mmol/L, about 80 mmol/L to 105 mmol/L, about 80 mmol/L to 100 mmol/L, about 80 mmol/L to 95 mmol/L, about 80 mmol/L to 90 mmol/L, about 80 mmol/L, about 85 mmol/L, about 90 mmol/L, about 95 mmol/L, about 100 mmol/L, about 110 mmol/L, about 115 mmol/L, or 120 mmol/L.

2.6 Glycine: In some embodiments of the present disclosure, the formulation (optionally) includes glycine as a protein stabilizer at a concentration of 50 to 75 mmol/L, for example, at a concentration of about 50 mmol/L to 73 mmol/L, about 50 mmol/L to 70 mmol/L, about 50 mmol/L to 67 mmol/L, about 50 mmol/L to 64 mmol/L, about 50 mmol/L to 60 mmol/L, about 50 mmol/L to 55 mmol/L, about 50 mmol/L, about 55 mmol/L, about 60 mmol/L, about 64 mmol/L, about 67 mmol/L, about 70 mmol/L, or 75 mmol/L.

2.7 Polysorbate 80: In some embodiments of the present disclosure, the formulation (optionally) includes polysorbate 80 as a surfactant at a concentration of about 0.02% to 0.03% (w/v, g/ml), for example, at a concentration of about 0.02% to 0.03%, about 0.02% to 0.04%, about 0.03% to 0.04%, about 0.03% to 0.05%, about 0.04% to 0.05%, about 0.02%, about 0.03%, about 0.04%, or about 0.05%

2.8 In a preferred embodiment of the present disclosure, the formulation may further include an antioxidant. Preferably the antioxidant is selected from the group consisting of methionine, sodium thiosulfate, catalase and platinum. The most preferred is methionine.

2.9 The antioxidant has a concentration typically ranging from about 0.01 mg/ml to about 50 mg/ml, about 0.01 mg/ml to about 10.0 mg/ml, about 0.01 mg/ml to about 5.0 mg/ml, about 0.01 mg/ml to about 1.0 mg/ml, or about 0.01 mg/ml to about 0.02 mg/ml. Preferably, the concentration of the antioxidant may be about 0.01 mg/ml, 0.02 mg/ml, 0.03 mg/ml, about 0.04 mg/ml, about 0.05 mg/ml, about 0.06 mg/ml, about 0.07 mg/ml, 0.08 mg/ml, 0.09 mg/ml, about 0.10 mg/ml, 0.11 mg/ml, 0.12 mg/ml, 0.13 mg/ml, about 0.14 mg/ml, about 0.15 mg/ml, about 0.16 mg/ml, about 0.17 mg/ml, 0.18 mg/ml, 0.19 mg/ml, about 0.20 mg/ml, about 0.25 mg/ml, 0.3 mg/ml, 0.4 mg/ml, 0.5 mg/ml, 0.6 mg/ml, 0.7 mg/ml, 0.8 mg/ml, 0.9 mg/ml, or 1.0 mg/ml.

2.10 In some embodiments of the present disclosure, the formulation includes methionine at a concentration of 10 mmol/L.

2.11 In an embodiment of the present disclosure, the antibody molecule is present in an amount of less than 120 mg/mL, preferably, in an amount of no more than 100 mg/mL.

In a preferred embodiment of the present disclosure, the antibody molecule may have a concentration ranging from about 0.1 to about 120 mg/ml. Preferably, the concentration of the antibody is about 0.5 mg/ml, about 1 mg/ml, about 2 mg/ml, about 2.5 mg/ml, about 3 mg/ml, about 3.5 mg/ml, about 4 mg/ml, about 4.5 mg/ml, about 5 mg/ml, about 5.5 mg/ml, about 6 mg/ml, about 6.5 mg/ml, about 7 mg/ml, about 7.5 mg/ml, about 8 mg/ml, about 8.5 mg/ml, about 9 mg/ml, about 9.5 mg/ml, about 10 mg/ml, about 11 mg/ml, about 12 mg/ml, about 13 mg/ml, about 14 mg/ml, about 15 mg/ml, about 16 mg/ml, about 17 mg/ml, about 18 mg/ml, about 19 mg/ml, about 20 mg/ml, about 21 mg/ml, about 22 mg/ml, about 23 mg/ml, about 24 mg/ml, about 25 mg/ml, about 26 mg/ml, about 27 mg/ml, about 28 mg/ml, about 29 mg/ml, about 30 mg/ml, about 31 mg/ml, about 32 mg/ml, about 33 mg/ml, about 34 mg/ml, about 35 mg/ml, about 36 mg/ml, about 37 mg/ml, about 38 mg/ml, about 39 mg/ml, about 40 mg/ml, about 41 mg/ml, about 42 mg/ml, about 43 mg/ml, about 44 mg/ml, about 45 mg/ml, about 46 mg/ml, about 47 mg/ml, about 48 mg/ml, about 49 mg/ml, about 50 mg/ml, about 51 mg/ml, about 52 mg/ml, about 53 mg/ml, about 54 mg/ml, about 55 mg/ml, about 56 mg/ml, about 57 mg/ml, about 58 mg/ml, about 59 mg/ml, about 60 mg/ml, about 70 mg/ml, about 80 mg/ml, about 90 mg/ml, about 100 mg/ml, about 110 mg/ml, or 120 mg/ml.

2.12 In some embodiments of the present disclosure, the concentration of the anti-IL-4 R$\alpha$ /IL-5 bispecific antibody is 100 mg/mL.

2.13 In some embodiments of the present disclosure, the formulation includes 100 mg/mL anti-IL-4 R$\alpha$ /IL-5

bispecific antibody, 20 mmol/L acetic acid-sodium acetate, 50 mmol/L glycine, 50 mmol/L arginine hydrochloride, 100 mmol/L sucrose and 0.05 w/v% of polysorbate 80, with pH of 5.0 to 5.2.

2.14 In some embodiments of the present disclosure, the formulation includes 100 mg/mL anti-IL-4 Rα /IL-5 bispecific antibody, 20 mmol/L acetic acid-sodium acetate, 50 mmol/L glycine, 50 mmol/L arginine hydrochloride, 100 mmol/L sucrose, 10 mmol/L methionine and 0.05 w/v% of polysorbate 80, with pH of 5.0 to 5.2.

2.15 In some embodiments of the present disclosure, the formulation includes 100 mg/mL anti-IL-4 Rα /IL-5 bispecific antibody, 10 mmol/L acetic acid-sodium acetate, 50 mmol/L glycine, 50 mmol/L arginine hydrochloride, 120 mmol/L sucrose and 0.05 w/v% of polysorbate 80, with pH of 5.0 to 5.2.

2.16 In some embodiments of the present disclosure, the formulation includes 100 mg/mL anti-IL-4 Rα /IL-5 bispecific antibody, 10 mmol/L acetic acid-sodium acetate, 75 mmol/L glycine, 25 mmol/L arginine hydrochloride, 120 mmol/L sucrose and 0.05 w/v% of polysorbate 80, with pH of 5.0 to 5.2.

2.17 In some embodiments of the present disclosure, the formulation includes 100 mg/mL anti-IL-4 Rα /IL-5 bispecific antibody, 20 mmol/L acetic acid-sodium acetate, 100 mmol/L arginine hydrochloride, 80 mmol/L sucrose and 0.02 w/v% of polysorbate 80, with pH of 5.0 to 5.2.

2.18 In some embodiments of the present disclosure, the formulation further includes one or more selected from preservatives and/or excipients.

2.19 In some embodiments of the present disclosure, the formulation may include a preservative. Preferably the preservative is selected from phenol, m-cresol, benzyl alcohol, benzalkonium chloride, benzalthonium chloride, phenoxyethanol and methyl paraben. Typically, a concentration of the preservative ranges from about 0.001 mg/ml to about 50 mg/ml, from about 0.005 mg/ml to about 15.0 mg/ml, from about 0.008 mg/ml to about 12.0 mg/ml or from about 0.01 mg/ml to about 10.0 mg/ml. Preferably, the concentration of the preservative can be 0.1 mg/ml, 0.2 mg/ml, 0.3 mg/ml, about 0.4 mg/ml, about 0.5 mg/ml, about 0.6 mg/ml, about 0.7 mg/ml, 0.8 mg/ml, 0.9 mg/ml, about 1.0 mg/ml, 2.0 mg/ml, 3.0 mg/ml, about 4.0 mg/ml, about 5.0 mg/ml, about 6.0 mg/ml, about 7.0 mg/ml, 8.0 mg/ml, 9.0 mg/ml, about 9.1 mg/ml, about 9.2 mg/ml, 9.3 mg/ml, 9.4 mg/ml, 9.5 mg/ml, 9.6 mg/ml, 9.7 mg/ml, 9.8 mg/ml, 9.9 mg/ml, or 10.0 mg/ml. Most preferably, the concentration of the preservative is about 0. 1 mg/ml or 9.0 mg/mL.

In some embodiments of the present disclosure, the composition includes no antioxidants.

In some embodiments of the present disclosure, the composition includes no preservatives.

3.1 Viscosity

Increased viscosity of protein formulations has negative effects in processing, e.g. processability of the liquid through drug delivery to the patient, e.g. at a high viscosity, that is, the liquid formulation no longer easily passes through the gauge of a needle, causing discomfort to the patient. Additionally, antibody formulations at a relatively high concentration but a suitably low viscosity are desired as a prerequisite for easy manufacturing, storage, and administration. The term "viscosity" as used herein, may be "kinematic viscosity" or "absolute viscosity." Commonly, kinematic viscosity is expressed in centistokes (cSt). The SI unit of kinematic viscosity is $mm^2/s$, which is 1 cSt. Absolute viscosity is expressed in units of centipoise (cP). The SI unit of absolute viscosity is the millipascal-second (mPa-s), where 1 cP=1 mPa.s.

Suitable pharmaceutical formulations according to the present disclosure have a viscosity of about less than 16 mPa.s, more preferably less than 7 mPa.s, and preferably 5.7 to 6.1 mPa.s.

3.2 Osmotic pressure

In some embodiments of the present disclosure, the formulation of the present disclosure is isotonic.

The term "isotonic" means that the formulation of interest has essentially the same osmotic pressure as human blood. In an embodiment, the isotonic formulation of the disclosure generally has an osmotic pressure in the range of 299 to 331 mOsm. Isotonicity can be measured using a vapor pressure or freezing point osmometer for example.

3.3 Stability

In some embodiments of the present disclosure, the formulations of the present disclosure are stable for at least 3, 6, 9, 12 months (e.g., at least 24, 30, 36 months), at a temperature of about 2 °C to about 25 °C (e.g., about 4 °C or 25 °C). In some embodiments, the antibody molecule keeps intact after storage in the formulation for at least 3, 6, 9, 12 months (e.g., at least 24, 30, 36 months), at a temperature of about 2 °C to about 25 °C (e.g., about 4 °C or 25 °C). For example, the antibody in the formulation retains at least 50%, 70%, 75%, 80%, 85%, 90%, 95%, 98% or up to 100% of a biological activity, e.g., binding activity, of the antibody molecule after storage at a temperature of about 2 °C to about 25 °C (e.g., about 4 °C or 25 °C). In some embodiments, the formulation includes less than 10%, 9%, 5%, 4%, 3%, 2%, 1% or less high molecular weight (HMW) species after storage in the formulation for at least 3, 6, 9, 12 months (e.g., at least 24, 30, 36 months), at a temperature of about 2 °C to about 25 °C (e.g., about 4 °C or 25 °C). In other embodiments, the formulation includes less than 10%, 9%, 5%, 4%, 3%, 2%, 1% or less low molecular weight (LMW) species after storage in the formulation for at least 3, 6, 9, 12 months (e.g., at least 24, 30, 36 months), at a temperature of about 2 °C to about 25 °C (e.g., about 4 °C or 25 °C).

In yet other embodiments, the formulation includes less than 10%, 9%, 8%, 7%, 6%, 5%, 4%, 3%, 2%, 1% or less acidic species after storage in the formulation for at least 3, 6, 9, 12 months (e.g., at least 24, 30, 36 months), at a temperature of about 2 °C to about 25 °C (e.g., about 4 °C or 25 °C). In yet other embodiments, the formulation includes less than 10%, 9%, 8%, 7%, 6%, 5%, 4%, 3%, 2%, 1% or less basic species after storage in the formulation for at least 3, 6, 9, 12 months (e.g., at least 24, 30, 36 months), at a temperature of about 2 °C to about 25 °C (e.g., about 4 °C or 25 °C). The HMW, LMW, acidic and basic species in the formulations can be detected using standard techniques, such as size exclusion-high performance liquid chromatography (SEC-HPLC) and the like as described herein.

In some embodiments, upon reconstitution of the lyophilized antibody formulation, the formulation retains the structure of at least 80%, 90%, 95% or higher of the antibody molecules compared to the formulation prior to lyophilization. The structure of antibody molecules is determined, for example, by binding assay, bioassay, or the ratio of HMW species to LMW species.

In some embodiments of the present disclosure, the content of monomer in the formulation solution decreases by no more than 5% after storage at about 37 °C for 14 days. The monomer refers to the dual-chain antibody molecule in the present disclosure.

In some embodiments of the present disclosure, under conditions of -20 °C, 5 °C, shaking, or freezing and thawing, the content of monomer in the sample changes by no more than 1.5%.

4. Products

The liquid formulation can be present in a product, such as a device, a syringe or a vial with an instruction manual. In certain embodiments, the syringe or a vial is made of glass, plastic, or a polymeric material, such as cyclic olefin polymer or copolymer. In other embodiments, the formulation can be present in an injectable device.

For example, a syringe, e.g., a prefilled syringe. The syringe may be adapted for individual administration, e.g., as a single vial system including an autoinjector (e.g., a pen-injector device), and/or an instruction manual. The formulation can be administered to a subject, e.g., a patient, by injection, e.g., peripheral administration.

In some embodiments of the present disclosure, a container including the aforementioned stable bispecific antibody formulation is provided.

In some embodiments of the present disclosure, the aforementioned container includes a syringe or bottle made of any one of glass, plastic, or polymeric materials.

In some embodiments of the present disclosure, a sealed package including any of the above stable bispecific antibody formulation or the container is provided.

In some embodiments of the present disclosure, a delivery system including the aforementioned stable antibody formulation is provided and is selected from a disposable syringe.

In some embodiments of the present disclosure, a medicament for treating diseases including the aforementioned stable antibody formulation is provided.

5. Application and treatment methods in diseases

In some embodiments of the present disclosure, provided is use of a stable antibody formulation in preparing a medicament for treating diseases. The diseases include asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

In some embodiments of the present disclosure, the medicament is a medicament administered by the intravenous or subcutaneous route.

In some embodiments of the present disclosure, provided is a method for treating diseases using any of the above antibody formulations. The diseases include asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

In some embodiments of the present disclosure, provided is use of glycine in preparing a stable antibody formulation, wherein the antibody molecule would still be present stably upon the concentration of the antibody molecule in the antibody formulation reaching 100 mg/mL.

In some embodiments of the present disclosure, provided is use of an antibody formulation in improving the stability of high-concentration antibodies in liquid formulations.

6. Preparation process

The present disclosure provides a method for preparing a stable antibody formulation, including: step 1) preparing an antibody molecule; and step 2) mixing a therapeutically effective amount of the antibody molecule with a tonicity agent, a buffer agent, a surfactant and/or other required components to form a stable solution. In another aspect, the present disclosure describes a method or process of preparing the formulations described herein. The method or process includes: expressing the antibody molecule in a cell culture; purifying the antibody molecule, e.g., by passing the antibody molecule through at least one of a chromatography or other similar purification step, an ultrafiltration/diafiltration steps; adjusting the concentration of the antibody molecule using a formulation buffer agent, e.g., to about 10 to 100 mg/mL, and adjusting pH of the formulation to about 4.5 to 5.3, e.g., 4.5, 4.6, 4.7, 4.8, 4.9, 5.0, 5.1, 5.2 or 5.3.

DEFINITIONS

The term "protein formulation" or "antibody formulation" refers to preparations which are in such form that permit the biological activity of the active ingredients to be unequivocally effective, and which contain no additional components which are toxic to the subjects to be administered with the formulation.

"Pharmaceutically acceptable" excipients (vehicles, additives) are those which can reasonably be administered to a mammal subject to provide an effective dose of the active ingredient employed. For example, the concentration of the excipient is also relevant for acceptability for injection.

A "stable" formulation is one in which the protein therein substantially retains its physical and/or chemical stability and/or biological activity upon storage. Various analytical techniques for measuring protein stability are available in the art and are reviewed in Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993), for example. Stability can be measured at a selected temperature for a selected time period. Furthermore, the formulation is stable following freezing (e.g. to -80°C) and thawing of the product.

A protein "retains its physical stability" in a biopharmaceutical formulation if it shows little to no change in terms of aggregation, precipitation and/or denaturation as observed by visual examination of color and/or clarity, or as measured by UV light scattering (for measuring visible aggregates) or size exclusion chromatography (SEC). SEC can be used to measure soluble aggregates that are not necessarily a precursor for visible aggregates. A protein "retains its chemical stability" in a biopharmaceutical formulation, if the chemical stability of the protein at a given time is such that the protein is considered to retain its biological activity as defined below. Chemical stability can be assessed by detecting and quantifying chemically altered forms of the protein. Chemical alteration may involve size modification (e.g. clipping) which can be evaluated, for example using SEC, SDS-PAGE and/or matrix-assisted laser desorption ionization/time-of-flight mass spectrometry (MALDI/TOF MS). Other types of chemical alteration include charge alteration (e.g. occurring as a result of deamidation) which can be evaluated, for example by ionexchange chromatography.

An antibody "retains its biological activity" in a pharmaceutical formulation, if the change of the biological activity of the antibody at a given time is within about 10% (within the assay error) of the biological activity exhibited when the pharmaceutical formulation was prepared, for example as determined in an antigen binding assay. Other assays for "biological activity" of antibodies are elaborated herein below.

Single-domain antibodies (sdAb, also called Nanobody or VHH by Ablynx, the developer) are well known to the skilled person. Single-domain antibodies are antibodies whose complementarity determining regions are part of a single-domain polypeptide. Single-domain antibodies thus include a single complementarity determining region (CDR)1, a single CDR2 and a single CDR3. Examples of single-domain antibodies are antibodies having a heavy chain only (i.e. antibodies that naturally do not include a light chain), single-domain antibodies derived from conventional antibodies, and engineered antibodies.

Single-domain antibodies may be derived from any species including mouse, human, camel, llama, goat, rabbit, and cattle. For example, naturally occurring VHH molecules can be derived from antibodies raised in Camelidae species, for example in camel, dromedary, alpaca and guanaco. Like an intact antibody, a single-domain antibody is able to bind selectively to a specific antigen. Single-domain antibodies may contain only the variable domain of an immunoglobulin chain having CDR1, CDR2 and CDR3 and framework regions.

As used herein the term "sequence homology" indicates the extent to which two (nucleotide or amino acid) sequences have identical residues at the same positions in an alignment, and is often expressed as a percentage. Preferably, homology is determined over the entire length of the sequences being compared. Thus, two copies of exactly the same sequence have 100% homology.

In the present disclosure, bispecific antibodies can also be obtained from sequences with high homology to the sequences of CDRs 1-3 disclosed in the present disclosure. In some embodiments, sequences with "at least 80% homology", or "at least 85% homology", "at least 90% homology", "at least 95% homology" or "at least 98% homology" to the sequences as set forth in SEQ ID NO: 1 to 6 can also achieve the purpose of the present disclosure.

In some embodiments, sequences with only one or a few amino acids replaced with other amino acids (for example, containing 1, 2, 3, 4, 5, 6, 7, 8, 9 or 10 conservative amino acid substitutions) as compared to the foregoing sequence can also achieve the purpose of the present disclosure. These variation forms include, but are not limited to, deletion, insertion and/or substitution of one or more (typically 1 to 50, preferably 1 to 30, more preferably 1 to 20, most preferably 1 to 10) amino acids, and addition of one or several (typically 20 or less, preferably 10 or less, more preferably 5 or less) amino acids at the C-terminus and/or N-terminus. Indeed, the skilled person may consider so-called "conservative" amino acid substitutions when determining the degree of sequence homology between two amino acid sequences or when determining the combination of CDR1, CDR2 and CDR3 in a single-domain antibody. In the case of substitutions, the substitutions will preferably be conservative amino acid substitutions. The conservative amino acid substitution can generally be described as an amino acid residue that is replaced by another amino acid residue of similar chemical structure and which has little or essentially no influence on the function, activity or other biological properties of the polypeptide. The conservative amino acid substitutions are commonly used in the art, for example, the conservative amino acid substitutions are substitutions in which one or a few amino acids within the following groups (a) - (d) is substituted by another or a few amino acids residue within the same group: (a) polar, negatively charged residues and their uncharged amides: Asp, Asn, Glu and Gln; (b) polar, positively charged residues: His, Arg and Lys; (c) aromatic residues: Phe, Tyr and Trp; (d) small aliphatic, nonpolar or slightly polar residues: Ala, Ser, Thr, Gly, Pro, Met, Leu, Ile, Val, Cys. Particularly preferred conservative amino acid substitutions are as follows: Asp into Glu; Asn into Gln or His; Glu into Asp; Gln into Asn; His into Asn or Gln; Arg into Lys; Lys into Arg, Gln; Phe into Met, Leu, Tyr; Trp into Tyr; Tyr into Phe, Trp; Ala into Gly or Ser; Ser into Thr; Thr into Ser; Gly into Ala or Pro; Met into Leu, Tyr or Ile; Leu into Ile or Val; Ile into Leu or Val; Val into Ile or Leu; Cys into Ser. In addition, those skilled in the art know that the inventiveness of single-domain antibodies is reflected in the CDR1-3 regions, and the framework sequences FRs 1-4 is not unchangeable. The sequences of FRs 1-4 can adopt the conservative sequence variants of the sequences disclosed in the present disclosure.

The present disclosure relates to the following aspects:

1. A stable antibody formulation, comprising a therapeutically effective amount of an antibody molecule, a tonicity agent, a buffer agent, and a surfactant, wherein the formulation has a pH of 4.5 to 5.5; the antibody molecule is capable of specifically binding to both IL-5 and IL-4Rα, and comprises at least two immunoglobulin variable domains comprising a first immunoglobulin variable domain capable of specifically binding to IL-4Rα molecule and a second immunoglobulin variable domain capable of specifically binding to IL-5.

2. The antibody formulation of item 1, wherein the formulation has a pH of 4.7 to 5.3.

3. The antibody formulation of item 1, wherein the first immunoglobulin variable domain is a first single-domain antibody molecule and comprises CDR1 as set forth in SEQ ID NO: 1, CDR2 as set forth in SEQ ID NO: 2, and CDR3 as set forth in SEQ ID NO: 3; and the second immunoglobulin variable domain is a second single-domain antibody molecule and comprise CDR1 as set forth in SEQ ID NO: 4, CDR2 as set forth in SEQ ID NO: 5, and CDR3 as set forth in SEQ ID NO: 6.

4. The antibody formulation of item 3, wherein the antibody molecule is a polypeptide fusion comprising no linker group between the first single-domain antibody molecule and the second single-domain antibody molecule.

5. The antibody formulation of item 3, wherein the antibody molecule is a polypeptide fusion and the first single-domain antibody molecule and the second single-domain antibody molecule of the polypeptide fusion are ligated via a biocompatible polymer as a linker group.

6. The antibody formulation of item 3, wherein the first single-domain antibody molecule and the second single-domain antibody molecule are ligated via a human IgG Fc region; the antibody molecule has a homodimer structure, and is named 4E9V10-2B3V2; each single-chain Fc fusion proteins is composed of a first immunoglobulin variable domain, a human IgG Fc region, and a second immunoglobulin variable domain.

7. The antibody formulation of item 6, wherein the human IgG Fc region is derived from human IgG1, IgG2, IgG3 or IgG4.

8. The antibody formulation of item 6, wherein the human IgG Fc region is derived from human IgG4 region; and the Fc region has an amino acid sequence as set forth in SEQ ID NO: 13, or at positions 116 to 356 of SEQ ID NO: 9.

9. The antibody formulation of item 1, wherein the first immunoglobulin variable domain has an amino acid sequence as set forth in SEQ ID NO: 7, and the second immunoglobulin variable domain has an amino acid sequence as set forth in SEQ ID NO: 8.

10. A stable antibody formulation, comprising a therapeutically effective amount of an antibody molecule, a tonicity agent, a buffer agent, and a surfactant, wherein the formulation has a pH of 4.5 to 5.5; and the antibody molecule comprises a fusion protein having an amino acid sequence as set forth in SEQ ID NO: 9 or 10.

11. The antibody formulation of item 1, wherein the buffer agent is an acetic acid-sodium acetate buffer, the tonicity agent comprises sucrose, and the surfactant comprises polysorbate.

12. The antibody formulation of item 11, further comprising a filler comprising glycine, and arginine hydrochloride.

13. The antibody formulation of item 11, wherein a concentration of acetic acid-sodium acetate is 10 to 20 mmol/L, and/or a concentration of arginine hydrochloride is 25 to 100 mmol/L, and/or a concentration of sucrose is 80 to 120 mmol/L, and/or a concentration of glycine is 50 to 75 mmol/L, and/or a concentration of polysorbate 80 is 0.02 to 0.05 w/v%.

14. The antibody formulation of item 1, further comprising methionine.

15. The antibody formulation of item 14, further comprising methionine at a concentration of 10 mmol/L.

16. The antibody formulation of item 1, wherein the antibody molecule is present in an amount of less than 120 mg/mL

17. The antibody formulation of item 1, wherein a concentration of the antibody molecule is about 0.01 to about 100 mg/ml.

18. The antibody formulation of item 1, comprising 100 mg/mL anti-IL-4 R$\alpha$/IL-5 bispecific antibody, 20 mmol/L acetic acid-sodium acetate, 50 mmol/L glycine, 50 mmol/L arginine hydrochloride, 100 mmol/L sucrose and 0.05 w/v% of polysorbate 80, with pH of 5.0 to 5.2.

19. The antibody formulation of item 2, comprising 100 mg/mL anti-IL-4 R$\alpha$/IL-5 bispecific antibody, 20 mmol/L acetic acid-sodium acetate, 50 mmol/L glycine, 50 mmol/L arginine hydrochloride, 100 mmol/L sucrose, 10 mmol/L methionine and 0.05 w/v% of polysorbate 80, with pH of 5.0 to 5.2.

20. The antibody formulation of item 1, comprising 100 mg/mL anti-IL-4 R$\alpha$/IL-5 bispecific antibody, 10 mmol/L acetic acid-sodium acetate, 50 mmol/L glycine, 50 mmol/L arginine hydrochloride, 120 mmol/L sucrose and 0.05 w/v% of polysorbate 80, with pH of 5.0 to 5.2.

21. The antibody formulation of item 1, comprising 100 mg/mL anti-IL-4 R$\alpha$/IL-5 bispecific antibody, 10 mmol/L acetic acid-sodium acetate, 75 mmol/L glycine, 25 mmol/L arginine hydrochloride, 120 mmol/L sucrose and 0.05 w/v% of polysorbate 80, with pH of 5.0 to 5.2.

22. The antibody formulation of item 1, comprising 100 mg/mL anti-IL-4 R$\alpha$/IL-5 bispecific antibody, 20 mmol/L acetic acid-sodium acetate, 100 mmol/L arginine hydrochloride, 80 mmol/L sucrose and 0.02 w/v% of polysorbate 80, with pH of 5.0 to 5.2.

23. The antibody formulation of item 1, further comprising one or more selected from preservatives and/or excipients.

24. The antibody formulation of item 1, wherein a content of monomer in the formulation decreases by no more than 5% after storage at about 37 °C for 14 days

25. The antibody formulation of item 1, under conditions of -20 °C, 5 °C, shaking, or freezing and thawing, a content of monomer in the sample changes by no more than 1.5%.

26. A container comprising the stable antibody formulation of any one of items 1-25.

27. The container of item 26, wherein the container comprises a syringe or bottle made of any one or more of glass, plastic, or polymeric materials.

28. A sealed package comprising the antibody formulation of any one of items 1-25 or a container of item 26.

29. A kit, comprising the antibody formulation of any one of items 1-25 and a container containing the antibody formulation of any one of items 1-25.

30. The antibody formulation according to any one of items 1-25 or the kit of item 29, for use in preparing a medicament for preventing and/or treating an autoimmune disease.

31. Use of the antibody formulation of any one of items 1-25 in preparing a medicament for treating a disease, wherein the disease comprises asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

32. The use of item 31, wherein the medicament is a medicament administered by an intravenous or subcutaneous

## EP 4 530 299 A1

route.

33. A method for treating a disease using the antibody formulation of any one of items 1-25, wherein the disease comprises asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

34. A delivery system comprising the stable antibody formulation of any one of claims 1-25, wherein the delivery system is selected from a disposable syringe.

35. A method for preparing the antibody formulation of any one of items 1-25, comprising: step 1) preparing an antibody molecule; and step 2) mixing a therapeutically effective amount of the antibody molecule with a tonicity agent, a buffer agent, a surfactant and/or other required components to form a stable solution.

36. A medicament for treating a disease, comprising the antibody formulation of any one of items 1-25, wherein the disease comprises asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

37. Use of glycine in preparing the antibody formulation of any one of items 1-25, wherein the antibody molecule would still be present stably upon a concentration of the antibody molecule in the antibody formulation reaching 100 mg/mL.

38. Use of an antibody formulation of any one of items 1-25 in improving the stability of a high-concentration antibody in liquid formulations.

Compared with existing technology, the antibody formulation of the present disclosure can specifically bind both IL-4Rα and IL-5. The concentration of the antibody molecule in the formulation during storage is about 0.01 mg/ml to 120 mg/mL. Within this concentration range, the antibody, especially at high antibody concentrations (e.g., 100 mg/mL), in the formulation retains its physical and chemical stability and keeps intact. The content of the monomer in the formulation solution will decrease by no more than 5% after storage at about 37 °C for 14 days. Under the conditions of -20 °C, 5 °C, shaking, or freezing and thawing, the content of the monomer in the sample changes by no more than 1.5%, which meets the application requirements of high-concentration antibody formulations.

BRIEF DESCRIPTION OF THE DRAWINGS

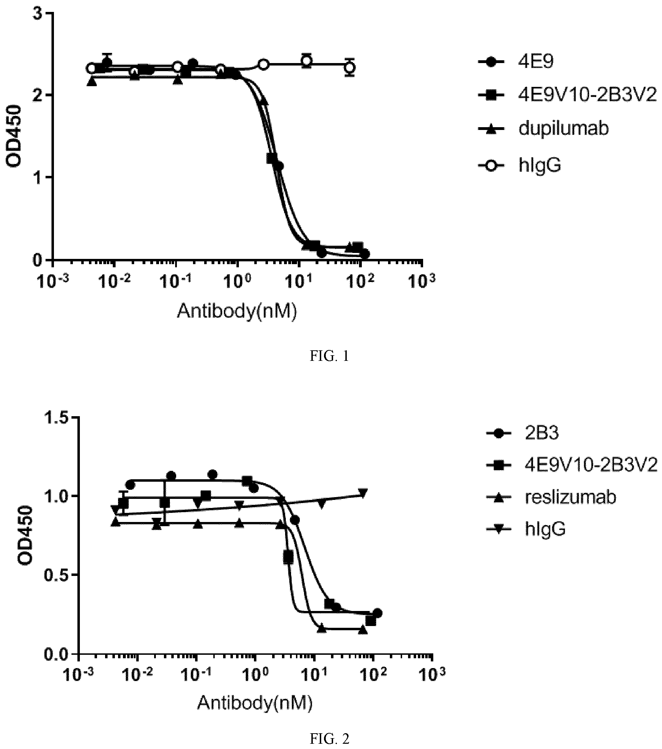

FIG. 1 shows the assay results (ELISA) of IL-4Rα/IL-5 bispecific antibody 4E9V10-2B3V2-1 blocking the binding of IL-4Rα to IL-4.

FIG. 2 shows the assay results (ELISA) of IL-4Rα/IL-5 bispecific antibody 4E9V10-2B3V2-1 blocking the binding of IL-5R to IL-5.

FIG. 3 shows the assay results of IL-4Rα/IL-5 bispecific antibody 4E9V10-2B3V2-1 neutralizing IL-4-induced TF-1 proliferation.

FIG. 4 shows the assay results of IL-4Rα/IL-5 bispecific antibody 4E9V10-2B3V2-1 neutralizing IL-13-induced TF-1 proliferation.

FIG. 5 shows the assay results of IL-4Rα/IL-5 bispecific antibody 4E9V10-2B3V2-1 neutralizing IL-5-induced TF-1 proliferation.

FIG. 6 is the dose-effect curve of sample B (4E9V10-2B3V2-2) neutralizing IL-4 and IL-5 (4:1) mixture-induced TF-1 proliferation.

FIG. 7 is the dose-effect curve of sample B (4E9V10-2B3V2-2) neutralizing IL-4 and IL-5 (4:1) mixture-induced TF-1 proliferation.

FIG. 8 is the dose-effect curve of sample B (4E9V10-2B3V2-2) neutralizing IL-4 and IL-5 (3:1) mixture-induced TF-1 proliferation.

FIG. 9 is the dose-effect curve of sample B (4E9V10-2B3V2-2) neutralizing IL-4 and IL-5 (3:1) mixture-induced TF-1 proliferation.

FIG. 10 is the dose-effect curve of sample B (4E9V10-2B3V2-2) neutralizing IL-4 and IL-5 (2:1) mixture-induced TF-1 proliferation.

FIG. 11 is the dose-effect curve of sample B (4E9V10-2B3V2-2) neutralizing IL-4 and IL-5 (2:1) mixture-induced TF-1 proliferation.

FIG. 12 is the dose-effect curve of sample B (4E9V10-2B3V2-2) neutralizing IL-4 and IL-5 (1:1) mixture-induced TF-1 proliferation.

FIG. 13 is the dose-effect curve of sample B (4E9V10-2B3V2-2) neutralizing IL-4 and IL-5 (1:1) mixture-induced TF-1 proliferation.

FIG. 14 is the dose-effect curve of sample B (4E9V10-2B3V2-2) neutralizing IL-4 and IL-5 (1:2) mixture-induced TF-1 proliferation.

FIG. 15 is the dose-effect curve of sample B (4E9V10-2B3V2-2) neutralizing IL-4 and IL-5 (1:2) mixture-induced TF-1 proliferation.

FIG. 16 is the dose-effect curve of sample B (4E9V10-2B3V2-2) neutralizing IL-4 and IL-5 (1:3) mixture-induced TF-1 proliferation.

FIG. 17 is the dose-effect curve of sample B (4E9V10-2B3V2-2) neutralizing IL-4 and IL-5 (1:3) mixture-induced TF-1 proliferation.

FIG. 18 is the dose-effect curve of sample B (4E9V10-2B3V2-2) neutralizing IL-4 and IL-5 (1:4) mixture-induced TF-1 proliferation.

FIG. 19 is the dose-effect curve of sample B (4E9V10-2B3V2-2) neutralizing IL-4 and IL-5 (1:4) mixture-induced TF-1 proliferation.

DETAILED DESCRIPTION

The present disclosure will be further described in detail below with reference to the Examples, so that those skilled in the art can implement it according to the description.

Example 1

Construction of Fc fusion antibody eukaryotic expression vector for anti-IL-4Rα/IL-5 bispecific single-domain antibody

For 4E9V10-2B3V2-1:

(1) A codon-optimized gene sequence (positions 1 to 345 of SEQ ID NO: 11) of anti-IL-4Rα humanized single-domain antibody (4E9V10) and a codon-optimized gene sequence (positions 1069 to 1434 of SEQ ID NO: 11) of anti-IL-5 humanized single-domain antibody (named 2B3V2) were synthesized through sequence synthesis and inserted into a vector RJK-V4-3 (obtained referring to the method disclosed in Chinese invention patent application no. CN202010576200.7 titled with "ANTI-IL-4RA SINGLE-DOMAIN ANTIBODY AS WELL AS APPLICATION AND DRUG"), respectively;

The vector RJK-V4-3 was obtained by the applicant through fusing the Fc segment of the heavy chain coding sequence of human IgG4 on the basis of a commercial vector pCDNA3.4 from Invitrogen (information for the vector can be found in https://assets.thermofisher.com/TFS-Assets/LSG/manuals/pcdna3_4_topo_ta_cloning_kit_man.pdf), that is, the vector contains the hinge region and CH2 and CH3 regions of the IgG4 heavy chain. The scheme was as follows.

(1) the restriction sites XbaI and AgeI on pcDNA3.4 were selected;
(2) multiple cloning site (MCS) and 6 × His tags were introduced through overlapping PCR at the 5' and 3' ends of the Fc fragment coding sequence respectively;
(3) the above fragment was amplified by PCR using a pair of primers each with XbaI and AgeI restriction sites;
(4) the pcDNA3.4 and recombinant DNA fragments in (3) were each digested using restriction endonucleases XbaI and AgeI;
(5) the digested vector was ligated with the fragment to be inserted under the action of T4 ligase, then the ligation product was transformed into E. coli for amplification, followed by sequencing for verification, obtaining the recombinant plasmid;
The Fc segment of the heavy chain coding sequence of the fused human IgG4 has a nucleotide sequence as shown at positions 346-1068 of SEQ ID NO: 11;

(2) the constructed recombinant eukaryotic expression vector was transformed into DH5α E. coli, which was cultured and subjected to plasmid maxiprep extraction for removal of endotoxin;

(3) the extracted plasmid was sequenced again for identification.

(4) After has been confirmed by identification, the eukaryotic expression vector for the anti-IL-4Rα/IL-5 bispecific single-domain antibody was constructed as follows. The eukaryotic expression vector, where the sequence of the anti-IL-4Rα antibody is located, was digested using restriction endonucleases XbaI and BamHI, the digested anti-IL-4Rα antibody was ligated to a eukaryotic expression vector containing the sequence of anti-IL-5 antibody and having the same restriction endonuclease sticky ends, and the ligated product was transformed and sequenced for identification. The clones confirmed by sequencing were subjected to plasmid maxiprep extraction for removal of endotoxin. The extracted plasmid was sequenced again for identification. The confirmed recombinant vector was prepared for subsequent transfection and expression in eukaryotic cells.

The anti-IL-4Rα/IL-5 bispecific antibody (each single chain of the bispecific antibody has an amino acid sequence as set forth in SEQ ID NO.9, and has a corresponding gene sequence as set forth in SEQ ID NO: 11) named 4E9V10-2B3V2 (specifically 4E9V10-2B3V2-1), includes three segments: 4E9V10-Fc segment-2B3V2, wherein 4E9V10 is located at the amino terminus (positions 1 to 115 of SEQ ID NO. 9), Fc segment is located at positions 116 to 356 of SEQ ID NO. 9, and 2B3V2 is located at the carboxyl terminus (positions 357 to 478 of SEQ ID NO. 9).

For 4E9V10-2B3V2-2:

(1) A codon-optimized gene sequence (positions 1 to 345 of SEQ ID NO: 12) of anti-IL-4Rα humanized single-domain antibody (4E9V10) and a codon-optimized gene sequence (positions 1069 to 1434 of SEQ ID NO: 12) of anti-IL-5 humanized single-domain antibody (named 2B3V2) were synthesized through sequence synthesis and inserted into a vector (obtained referring to the method disclosed in Chinese invention patent application no. CN202010576200.7 titled with "ANTI-IL-4RA SINGLE-DOMAIN ANTIBODY AS WELL AS APPLICATION AND DRUG"), respectively; the vector here was basically the same as the aforementioned RJK-V4-3, except that the nucleotide sequence of the Fc segment of the heavy chain coding sequence of the human IgG4 to be fused was replaced with the nucleotides as shown at positions 346-1068 of SEQ ID NO. 12;

(2) the constructed recombinant eukaryotic expression vector was transformed into DH5α E. coli, which was cultured and subjected to plasmid maxiprep extraction for removal of endotoxin;

(3) the extracted plasmid was sequenced again for identification.

(4) After has been confirmed by identification, the eukaryotic expression vector for the anti-IL-4Rα/IL-5 bispecific single-domain antibody was constructed as follows. The eukaryotic expression vector, where the sequence of the anti-IL-4Rα antibody is located, was digested using restriction endonucleases XbaI and BamHI, the digested anti-IL-4Rα antibody was ligated to a eukaryotic expression vector containing the sequence of the anti-IL-5 antibody and having the same restriction endonuclease sticky ends, and the ligated vector was transformed and sequenced for identification. The clones confirmed by sequencing were subjected to plasmid maxiprep extraction for removal of endotoxin. The extracted plasmid was sequenced again for identification. The confirmed recombinant vector was prepared for subsequent transfection and expression in eukaryotic cells.

The anti-IL-4Rα/IL-5 bispecific antibody (each single chain of the bispecific antibody has an amino acid sequence as set forth in SEQ ID NO: 10, and has a corresponding gene sequence as set forth in SEQ ID NO: 12) named 4E9V10-2B3V2 (specifically 4E9V10-2B3V2-2), includes three segments: 4E9V10-Fc segment-2B3V2, wherein 4E9V10 is located at the amino terminus (positions 1 to 115 of SEQ ID NO. 10), Fc segment is located at positions 116 to 356 of SEQ ID NO. 10, and 2B3V2 is located at the carboxyl terminus (positions 357 to 478 of SEQ ID NO. 10).

Example 2

The anti-IL-4Rα/IL-5 bispecific single-domain antibody was expressed in suspension ExpiCHO-S cells based on the assay method referring to Chinese invention patent no. CN202010576200.7 titled with "ANTI-IL-4RA SINGLE-DOMAIN ANTIBODY AS WELL AS APPLICATION AND DRUG". The bispecific antibody 4E9V10-2B3V2-1 (sample A) and the bispecific antibody 4E9V10-2B3V2-2 (sample B) were expressed and obtained, respectively.

Example 3

The anti-IL-4Rα/IL-5 bispecific single-domain antibody was expressed in suspension 293F cells based on the assay method referring to Chinese invention patent no. CN202010576200.7 titled with "Anti-IL-4Rα single-domain antibody as well as application and drug". The bispecific antibody 4E9V10-2B3V2-1 (sample A) and the bispecific antibody 4E9V10-2B3V2-2 (sample B) were respectively expressed.

Example 4

The anti-IL-4Rα/IL-5 bispecific single-domain antibody was purified based on the assay method referring to Chinese invention patent no. CN202010576200.7 titled with "ANTI-IL-4RA SINGLE-DOMAIN ANTIBODY AS WELL AS APPLICATION AND DRUG". The bispecific antibody 4E9V10-2B3V2-1 (sample A) and the bispecific antibody 4E9V10-2B3V2-2 (sample B) were obtained, respectively.

The sample A and the sample B used in all the following Examples were obtained by expression of the corresponding recombinant vector in suspension 293F cells and purification in Example 4.

Example 5

Blocking assay of receptor-ligand binding by bispecific antibody 4E9V10-2B3V2-1 (sample A)

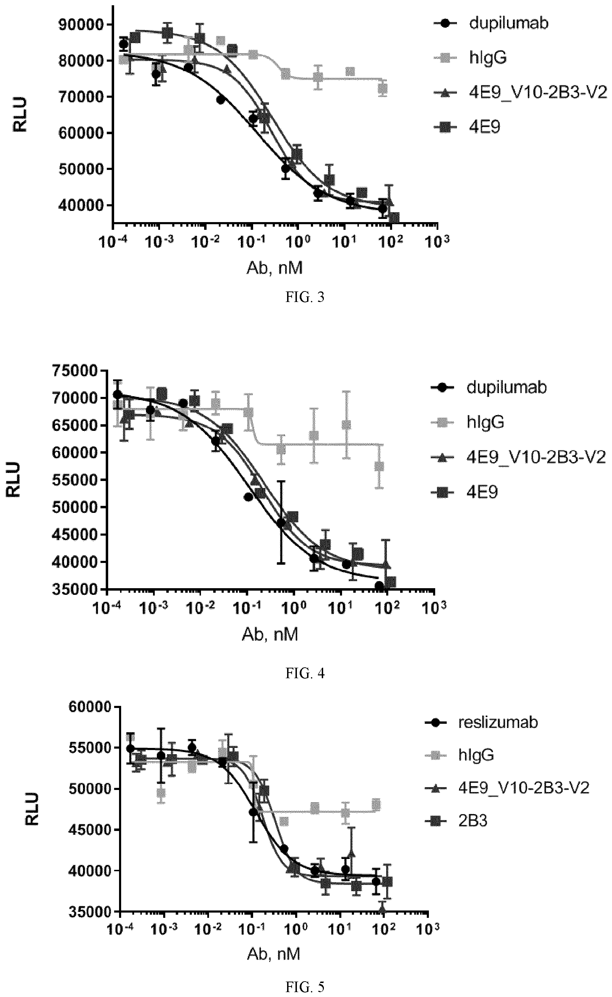

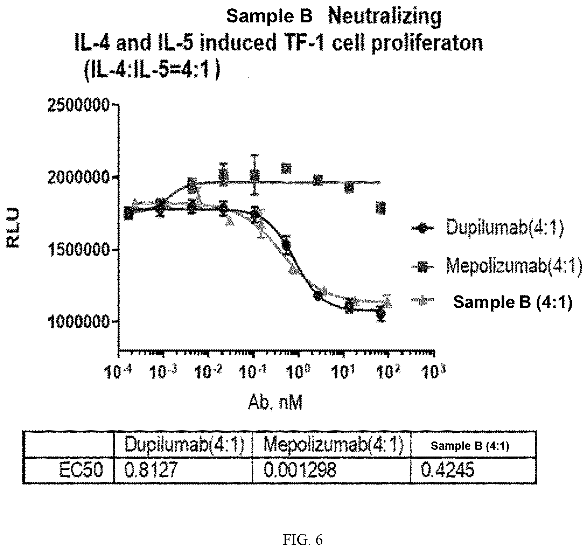

(1) The receptor protein (IL-4Rα or IL-5R) was diluted to 1 μg/ml with a protein dilution buffer, and coated a plate overnight at 4°C.
(2) The plate was washed and blocked with 5% skim milk at 37 °C.
(3) A biotin-conjugated ligand protein (IL-4 or IL-5) was 2-fold diluted with respect to its EC80 concentration, and the antibody was 2-fold diluted with respect to its initial concentration, followed by 5-fold gradient dilution. The ligand protein, and the diluted antibody (dupilumab, 4E9V10-2B3V2-1, 4E9-V0 or 2B3, reslizumab) and hIgG respectively, were transferred at 1:1 into a new dispensing plate and mixed well. 4E9V0 is the antibody 4E9 from CN202010576200.7 titled with "ANTI-IL-4RA SINGLE-DOMAIN ANTIBODY AS WELL AS APPLICATION AND DRUG"; and 2B3 is the single-domain antibody 2B3 (non-humanized) from CN202010843501.1 titled with "IL-5 BINDING MOLECULES AND PREPARATION METHODS AND APPLICATIONS THEREOF". Reslizumab and dupilumab used in this Example and Examples 6-8 were Tabs made by the applicant. The reslizumab used in this example and Examples 6-8 was prepared based on the method as described in CN202010843501.1 titled with "IL-5 BINDING MOLECULES AND PREPARATION METHODS AND APPLICATIONS THEREOF". The dupilumab used in this example and Examples 6-8 was prepared based on the method as described in CN202010576200.7 titled with "ANTI-IL-4RA SINGLE-DOMAIN ANTIBODY AS WELL AS APPLICATION AND DRUG".
(4) The plate was washed and, and the diluted ligand protein/antibody mixture was transferred into the ELISA plate in duplicate, and incubated at 37°C.
(5) The plate was washed, the diluted Streptavidin [HRP] was added and the mixture was incubated at 37 °C.
(6) The plate was washed, single-component TMB was added for color development at room temperature in the dark.
(7) Upon adding a stopping solution, the OD450 was immediately read on a microplate reader and EC50 was calculated by plotting. 4E9-V0 2B3 which is a non-humanized anti-IL-5 single-domain antibody, dupilumab, reslizumab, and hIgG were used as controls, and the results are shown in Tables 1 and 2 and FIGs. 5 and 6, respectively.

Table 1 Assay results of EC50 of bispecific antibodies blocking the binding of IL-4 to IL-4Rα

|  | 4E9V0 | 4E9V10-2B3V2-1 | dupilumab | hIgG |
|---|---|---|---|---|
| EC50(nM) | 4.447 | 3.653 | 4.321 | 2.038 |

Table 2 Assay results of EC50 of bispecific antibodies blocking the binding of IL-5 to IL-5R

|  | 2B3 | 4E9V10-2B3V2-1 | reslizumab | hIgG |
|---|---|---|---|---|
| EC50(nM) | 6.911 | 3.612 | 6.208 | 27387599489 |

The results show that the humanized bispecific antibody was non-attenuated and slightly advantageous in blocking IL-4/IL-4Rα or IL-5/IL-5R receptor-ligand binding than the non-humanized antibody. Compared with the corresponding commercial drugs, the humanized bispecific antibody has almost the same and slightly advantageous blocking ability.

Example 6

Assay for IL-4-induced or IL-13-induced TF1 cell proliferation by bispecific antibody 4E9V10-2B3V2-1 (sample A)

(1) TF-1 cells passaged 3-4 times after recovery were plated in a 96-well plate at 10000 cells/well.

(2) a solution of 10 μg/mL Tabs or the bispecific antibodies were formulated, respectively, and subjected to 5-fold gradient dilution.

(3) The Tab antibodies (obtained referring to a method disclosed in the Chinese invention patent application no. CN202010576200.7 titled with "ANTI-IL-4RA SINGLE-DOMAIN ANTIBODY AS WELL AS APPLICATION AND DRUG") and the sample A both subjected to gradient dilution were mixed with IL-4 or IL13 at EC80 concentration obtained referring to the proliferation assay disclosed in the Chinese invention patent application no. CN202010576200.7(titled with "Anti-IL-4Rα single-domain antibody as well as application and drug") at 1:1 to prepare a mixed solution.

(4) The mixed solution in the previous step was added into cell culture wells in an equal volume to the cell culture solution.

(5) After incubation for 72 h, the cell viability was detected with a luminescence cell viability assay kit.

(6) The EC50 concentrations of different antibodies for neutralizing IL-4-induced or IL-13-induced TF-1 cell proliferation was calculated according to the assay results.

The assay results are shown in Table 3, Table 4, FIG. 3 and FIG. 4.

Table 3 Assay results of bispecific single-domain antibodies neutralizing IL-4-induced TF-1 proliferation

|  | 4E9V0 | 4E9V10-2B3V2-1 | dupilumab | hIgG |
|---|---|---|---|---|
| EC50(nM) | 0.2812 | 0.2618 | 0.1297 | 0.3750 |

Table 4 Assay results of bispecific single-domain antibodies neutralizing IL-13-induced TF-1 proliferation

|  | 4E9V0 | 4E9V10-2B3V2-1 | dupilumab | hIgG |
|---|---|---|---|---|
| EC50(nM) | 0.2291 | 0.2154 | 0.1783 | 0.1312 |

Example 7

Assay for human recombinant IL-5 protein-induced TF1 cell proliferation and tool antibody (Tab) neutralizing proliferation

A. Assay for human recombinant IL-5 protein-induced TF1 cell proliferation:

(1) TF-1 cells passaged 3-4 times after recovery were plated in a 96-well plate at 10000 cells/well.

(2) A solution of the human IL-5 protein with a maximum concentration of 500 ng/mL was formulated, and subjected to 5-fold gradient dilution.

(3) The IL-5 protein solution subjected to gradient dilution was added into cell culture wells in an equal volume to the cell culture solution.

(4) After incubation for 72 h, the cell viability was detected with a luminescence cell viability assay kit.

(5) The EC80 concentration for IL-5-induced TF-1 cell proliferation was calculated according to the assay results, and the calculated result was 2.96 ng/mL.

B. Assay for Tab neutralizing human IL-5-induced TF1 cell proliferation

(1) TF-1 cells passaged 3-4 times after recovery were plated in a 96-well plate at 10000 cells/well.

(2) A solution of 10 μg/mL Tab was formulated, and subjected to 5-fold gradient dilution.

(3) The Tab subjected to gradient dilution was mixed with the IL-5 at the EC80 concentration obtained in the proliferation assay at 1:1 to prepare a mixed solution.

(4) The mixed solution was added into cell culture wells in an equal volume to the cell culture solution.

(5) After incubation for 72 h, the cell viability was detected with a luminescence cell viability assay kit.

(6) The EC50 concentration of the Tab neutralizing the IL-5-induced TF-1 cell proliferation was calculated according to the assay results.

Example 8

Assay for humanized bispecific single-domain antibody 4E9V10-2B3V2-1 neutralizing IL-5-induced TF1 cell proliferation.

(1) TF-1 cells passaged 3-4 times after recovery were plated in a 96-well plate at 10000 cells/well.
(2) A solution of 10 μg/mL Tab or the aforementioned 4E9V10-2B3V2-1 antibody were formulated, respectively, and subjected to 5-fold gradient dilution.
(3) The Tab and 4E9V10-2B3V2-1 antibodies subjected to gradient dilution were mixed at 1:1 respectively with the IL-5 protein at the EC80 concentration obtained in Example 7 to prepare a mixed solution.
(4) The mixed solution was added into cell culture wells in an equal volume to the cell culture solution.
(5) After incubation for 72 h, the cell viability was detected with a luminescence cell viability assay kit.
(6) The EC50 concentrations of different antibodies neutralizing the IL-5-induced TF-1 cell proliferation were calculated according to the assay results, and the assay results are shown in Table 5 and FIG. 5.

Table 5 Assay results of bispecific antibodies neutralizing IL-5-induced TF-1 cell proliferation

|  | reslizumab | hIgG | 4E9V10-2B3V2-1 | 2B3 |
|---|---|---|---|---|
| EC50(nM) | 0.1221 | 0.1080 | 0.1753 | 0.3362 |

The results show that the humanized bispecific antibody was non-attenuated and slightly advantageous in neutralizing IL-5-induced TF-1 cell proliferation than the non-humanized antibody. Compared with the corresponding commercial drugs, the humanized bispecific antibody has almost the same blocking ability.

Example 9

Affinity kinetic assay for the humanized bispecific antibody 4E9V10-2B3V2-1 was carried out as follows.

(1) Preparation of an SD buffer: an appropriate amount of bovine serum albumin and Tween 20 were dissolved in 1 x PBS (pH 7.4), so that the mass (or volume) fractions of the bovine serum albumin and the Tween 20 were 0.1% and 0.02% respectively. The aforementioned 4E9V10-2B3V2-1 antibody was formulated into the SD buffer to a concentration of 10 μg/mL.
(2) Preparation of an antigen working solution: an antigen was firstly formulated to 200 nM into the SD buffer, and then subjected to 2-fold gradient dilution. A total of 5 concentration gradients were set, in addition to the SD buffer as a blank control.
(3) Preparation of a regenerating solution: an appropriate amount of a 0.1 M glycine stock solution was ten-fold diluted into deionized water and mixed well to obtain the regenerating solution.

Assay steps: Octet 96 and Data Acquisition software in the supporting computer were run. The bottom and sides of an acquisition probe were cleaned using a lens tissue with an appropriate amount of 75% ethanol, and the instrument was preheated for 15 min or more. Sensor pre-wetting: before the assay, the Sensor was soaked in the SD buffer for 10 min or more for later use. Then, the machine procedure was set according to baseline→antibody→baseline→antigen binding→antigen dissociation-sensor regeneration for assay operation. The assay results are shown in Table 6.

Table 6 Assay results

|  | Binding antigen | KD(M) | Ka(1/Ms) | Ka Error | Kd(1/s) | Kd Error | $R^2$ |
|---|---|---|---|---|---|---|---|
| 4E9-V10-2B3V2-1 | IL-4Rα | 2.33E-10 | 1.98E+05 | 1.24E+03 | 4.60E-05 | 6.73E-06 | 0.994 |
| 4E9-V10-2B3V2-1 | IL-5 | 6.63E-10 | 2.13E+05 | 1.29E+03 | 1.41E-04 | 5.84E-06 | 0.9905 |

The results show that the affinity of the bispecific antibody targeting IL4Rα and IL-5 was determined with two antigens respectively, and the corresponding affinities were both below 1 nM.

Example 10

A proliferation neutralization assay was established in this experiment to determine the activity of 4E9V10-2B3V2-2 (sample B) in neutralizing mixed human IL-4 and IL-5 induced TF-1 cell proliferation. For convenience, 4E9V10 -2B3V2- 2 will be referred to as sample B in subsequent descriptions.

Table 7 Information for positive control sample

| Sample name | Dupilumab | Mepolizumab |
|---|---|---|
| Manufacturer/Supplier | Sanofi Winthrop Industrie | GlaxoSmithKline Trading Services Ltd. |
| Lot No. | OL193F | 2T2P |
| Properties | a solution varying from transparent to slightly milky white, or from colorless to light yellow solution, without visible particles | a solution varying from transparent to milky white, or from colorless to light yellow or to light brown |
| Concentration | 150 mg/ml | 100 mg/ml |
| Storage Conditions | 2 to 8 °C, protected from light, sealed, and away from freezing. | 2 to 8 °C, protected from light, sealed, and away from freezing. |

Table 8 Information for negative control sample

| Sample name | Human IgG1, kappa Isotype Control |
|---|---|
| Manufacturer/Supplier | Sino Biological Inc. |
| Lot No. | HGIK |
| Aggregation | <5% aggregation |
| Concentration | >1 mg/ml |
| Purity | >95% |
| Endotoxin | <3 EU/mg |
| Storage Conditions | -20 °C |

Table 9 Information for sample to be tested

| Sample name | Sample B (4E9V10-2B3V2-2) |
|---|---|
| Batch No. | 20210317 |
| Concentration | 1.99 mg/ml |
| Storage Conditions | -80 °C |

Formulation method of samples

Test buffer: test medium, RPMI-1640 + 10% FBS, stored at 2 to 8 °C for later use.

Human IL-4 preparation: Human IL-4 was dissolved in sterile ultrapure water to 100 $\mu$g/ml. The prepared protein was stored at -80 °C for later use.

Human IL-5 preparation: Human IL-5 was dissolved in sterile ultrapure water to 100 $\mu$g/ml. The prepared protein was stored at -80 °C for later use.

3. Test methods and procedures

3.1 Cell culture (TF-1)

Cell culture medium: RPMI-1640 + 10%FBS + 2ng/ml GM-CSF, passage density was $3E^4$ to $7E^5$.

3.2 Preparation of cell suspension: an appropriate amount of TF-1 cells that were in good growth status and have a density of $1\times10^5$ to $4\times10^5$ cells/ml were put into a 15ml centrifuge tube, centrifuged at 1200 r for 5 minutes. The supernatant was discarded, and the precipitation was resuspended in 1 to 2 ml of Assay Buffer.

3.3 Cell counting and seeding: the cells were counted in the resuspended cell suspension, adjusted to a cell density of $2\times10^5$ cells/ml, and inoculated at 50 $\mu$l per well, or $1\times10^4$ cells/well, in a 96-well cell culture plate with transparent bottom and white edges.

3.4 IL-4 and IL-5 were mixed in proportion (4:1, 3:1, 2:1, 1:1, 1:2, 1:3, 1:4) to a total protein concentration of 100 $\mu$g/ml. 100 $\mu$g/ml mixed proteins were diluted with test buffer to 3.11 ng/ml, 2.6928 ng/ml, 3.1532 ng/ml, 5.184 ng/ml, 15.568 ng/ml, 6.84 ng/ml, and 3.9116 ng/ml, respectively, for later use. The controls or samples were diluted with test buffer

to $4 \times 10$ µg/ml, and then five-fold serial diluted in a 9-concentration gradient, and the 10th well was 0 µg/ml. Each concentration was set in duplicate. The mixture of IL-4 and IL-5 in each ratio was mixed with the diluted sample at 1:1.

3.5 The above-mixed sample at 50 µl/ well was added into the 96-well plate where the cells are plated.

3.6 The 96-well plate was placed in a cell culture incubator at 37°C and 5% $CO_2$ and incubated for 72 hours.

3.7 The cell plate was taken from the incubator, added with 100 µl CellTiter-Glo per well, incubated at room temperature for 15 minutes in the dark, and measured for the cell viability in each well with a multifunctional microplate reader.

In this assay, Dupilumab, Mepolizumab and Dupilumab + Mepolizumab (mixed at 1:1) were used as positive controls, Human IgG as a negative control, and sample B (4E9V10-2B3V2-2) as a sample to be tested. Each sample was set in duplicate. The samples with an initial concentration of 4*10 µg/mL were diluted in 5-fold gradient dilution to 9 concentrations. The last duplicate wells in each group were 0 µg/ml concentration. In a 96-well cell plate, the concentration in column 2 was 10 µg/ml, the concentration in column 3 was 2 µg/ml, the concentration in column 4 was 0.4 µg/ml, the concentration in column 5 was 0.08 µg/ml, and the concentration in column 6 was 0.016 µg/ml, the concentration in column 7 was 0.0032 µg/ml, the concentration in column 8 was 0.00064 µg/ml, the concentration in column 9 was 0.000128 µg/ml, the concentration in column 10 was 0.0000256 µg/ml, and the concentration in column 11 was 0 µg/ml. The IL-4 and IL-5 mixed in a proportion (4:1, 3:1, 2:1, 1:1, 1:2, 1:3, 1:4) were formulated to 3.11 ng/ml, 2.6928 ng/ml, 3.1532 ng/ml, 5.184 ng/ml, 15.568 ng/ml, 6.84 ng/ml, and 3.9116 ng/ml for later use, and mixed with the above samples at 1:1. Then the mixture and cells were mixed at a volume ratio of 1:1 and incubated in a $CO_2$ incubator for 72 hours (at 37 °C and 5% $CO_2$). Finally, a multifunctional microplate reader was used for reading, and the data was processed to draw a conclusion.

Four-parameter nonlinear regression curve fitting was performed using GraphPad Prism with the molar concentrations as the abscissa and relative light unit (RLU) as the ordinate to obtain EC50 and dose-effect curves. The dose-effect curves are shown in Figs. 6 to 19.

4. Main instruments and reagents

Table 10 List of Main instruments

| Instrument | Model | Producer |
|---|---|---|
| Multifunctional microplate reader | VICTOR Nivo | PerkinElmer |
| Biological safety cabinet | Bsc-1304IIA2 | Suzhou Antai Airtech Co., Ltd |
| Low speed cell centrifuge | DM0412 | SCILOGEX |
| Carbon dioxide incubator | CCL-170B-8 | ESCO |

Table 11 Main reagent list

| names of Consumables and reagents | Producer | Lot No. |
|---|---|---|
| RPMI-1640 | Gibco | 61870-127 |
| 96-well cell plate | CORNING | 3917 |
| FBS | Gibco | 10091-148 |
| GM-CSF | sigma | G5035 |
| Human IL-5 | Novoprotein | CI59 |
| Human IL-4 | Novoprotein | CX03 |
| CellTiter-Glo | Promega | G7573 |

5. Summary of results

Table 12 Summary of test results

| IL-4:IL-5 | Analytical standards | Dupilumab (Plate 1) | Mepolizumab (Plate 1) | Sample B (Plate 1) | Sample B (Plate 2) | Dupilumab+ Mepolizumab (Plate 2) | hIgG (Plate 2) |
|---|---|---|---|---|---|---|---|
| 4:1 | EC50 (nM) | 0.8127 | 0.001298 | 0.4245 | 0.3212 | 0.8250 | 0.1051 |
| | Window (times) | 1.77 | 1.02 | 1.59 | 1.78 | 1.68 | 1.01 |
| | | Dupilumab (Plate 3) | Mepolizumab (Plate 3) | Sample B (Plate 3) | Sample B (Plate 4) | Dupilumab+ Mepolizumab (Plate 4) | hIgG (Plate 4) |
| 3:1 | EC50 (nM) | 0.4481 | 0.001322 | 0.2429 | 0.2186 | 0.9652 | 0.0009147 |
| | Window (times) | 1.83 | 1.05 | 1.53 | 1.60 | 1.67 | 1.02 |
| | | Dupilumab (Plate 5) | Mepolizumab (Plate 5) | Sample B (Plate 5) | Sample B (Plate 6) | Dupilumab+ Mepolizumab (Plate 6) | hIgG (Plate 6) |
| 2:1 | EC50 (nM) | 0.4519 | 2.936 | 0.2402 | 0.2127 | 1.279 | N.A. |
| | Window (times) | 1.60 | 0.99 | 1.55 | 1.63 | 1.76 | 0.99 |
| | | Dupilumab (Plate 7) | Mepolizumab (Plate 7) | Sample B (Plate 7) | Sample B (Plate 8) | Dupilumab+ Mepolizumab (Plate 8) | hIgG (Plate 8) |
| 1:1 | EC50 (nM) | 0.5701 | 14.89 | 0.3692 | 0.3709 | 1.733 | 14.70 |
| | Window (times) | 1.77 | 1.11 | 1.71 | 1.70 | 1.92 | 1.07 |
| | | Dupilumab (Plate 9) | Mepolizumab (Plate 9) | Sample B (Plate 9) | Sample B (Plate 10) | Dupilumab+ Mepolizumab (Plate 10) | hIgG (Plate 10) |
| 1:2 | EC50 (nM) | 0.8986 | 0.001038 | 0.5947 | 0.7358 | 1.960 | 0.002641 |
| | Window (times) | 1.68 | 1.06 | 1.58 | 1.64 | 1.62 | 0.99 |
| | | Dupilumab (Plate 11) | Mepolizumab (Plate 11) | Sample B (Plate 11) | Sample B (Plate 12) | Dupilumab+ Mepolizumab (Plate 12) | hIgG (Plate 12) |
| 1:3 | EC50 (nM) | 0.3429 | 0.001468 | 0.1523 | 0.1077 | 0.8805 | 0.001926 |
| | Window (times) | 1.53 | 1.01 | 1.50 | 1.74 | 1.76 | 0.95 |
| | | Dupilumab (Plate 13) | Mepolizumab (Plate 13) | Sample B (Plate 13) | Sample B (Plate 14) | Dupilumab+ Mepolizumab (Plate 14) | hIgG (Plate 14) |
| 1:4 | EC50 (nM) | 0.1562 | 1.105 | 0.07883 | 0.04987 | 0.3949 | 0 |
| | Window (times) | 1.58 | 1.14 | 1.50 | 1.67 | 1.79 | 1.03 |

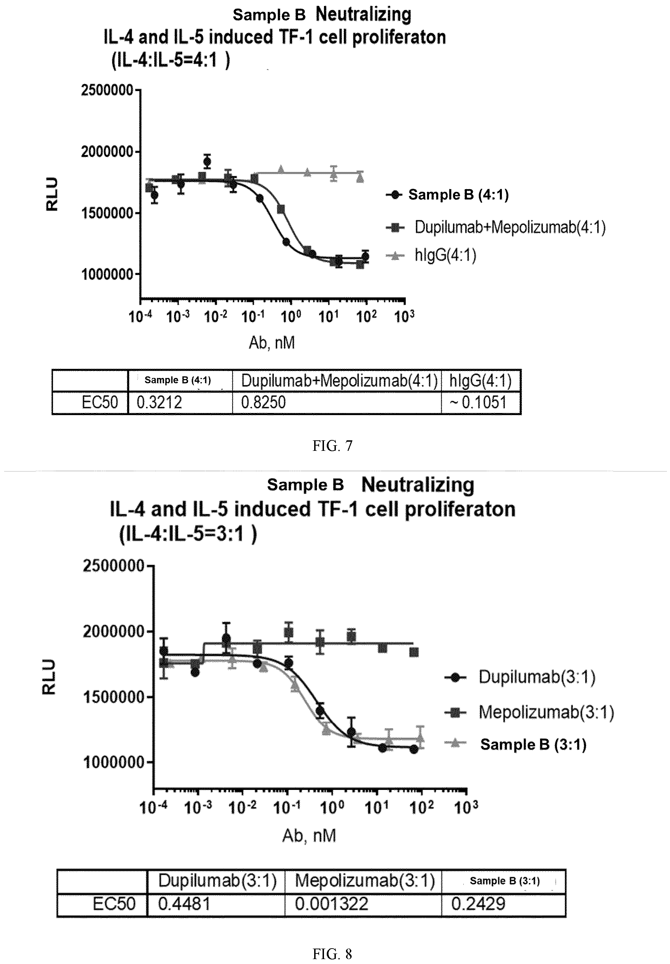

The results show that: when IL4 : IL5 = 4 : 1, in plate 1, the test window of positive control Dupilumab was 1.77 times, and the EC50 was 0.8127 nM; the test window of Mepolizumab was 1.02 times, and the EC50 was 0.001298 nM; the test window of sample B bivalent antibody was 1.59 times, and the EC50 was 0.4245 nM; in plate 2, the test window of sample B was 1.78 times, and the EC50 was 0.3212 nM; the window of control Dupilumab + Mepolizumab was 1.68 times, and the EC50 was approximately 0.8250 nM; the window of negative control human IgG was 1.01 times, and EC50 was approximately 0.1051 nM. The fitting curves based on the above data are shown in FIGS. 6 and 7.

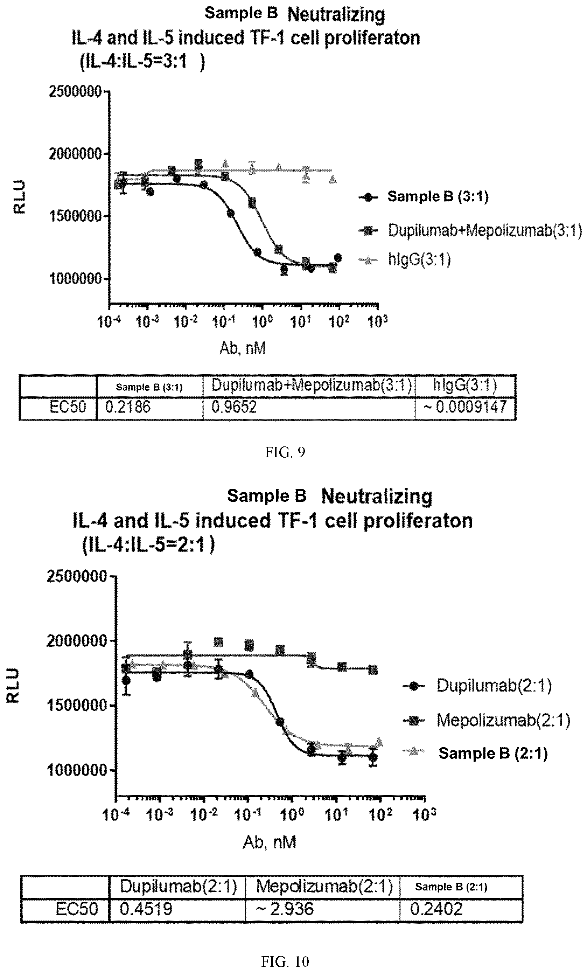

When IL4 : IL5 = 3 : 1, in plate 3, the test window of positive control Dupilumab was 1.83 times, and the EC50 was 0.4481 nM; the test window of Mepolizumab was 1.05 times, and the EC50 was 0.001322 nM; The test window of sample B bivalent antibody was 1.53 times, and the EC50 was 0.2429 nM; in plate 4, the window of sample B bivalent antibody was 1.60 times, and the EC50 was 0.2186 nM; the window of positive control Dupilumab + Mepolizumab was 1.67 times, and the EC50 was about 0.9652 nM; the window of negative control human IgG was 1.02 times, and EC50 was about 0.0009147 nM. The fitting curves based on the above data are shown in FIGS. 8 and 9.

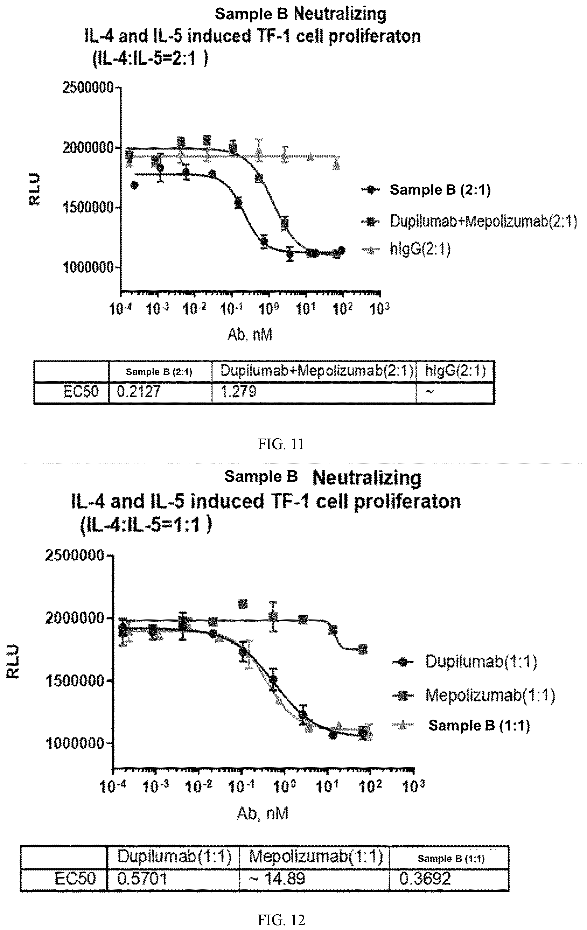

When IL4 : IL5 = 2 : 1, in plate 5, the test window of positive control Dupilumab was 1.60 times, and the EC50 was 0.4519 nM; the test window of Mepolizumab was 0.99 times, and the EC50 was 2.936 nM; the test window of sample B bivalent antibody was 1.55 times, and the EC50 was 0.2402 nM; in plate 6, the window of sample B bivalent antibody was 1.63 times, and the EC50 was 0.2127 nM; the window of positive control Dupilumab + Mepolizumab was 1.76 times, and the EC50 was about 1.279 nM; the window of negative control human IgG was 0.99 times, and EC50 cannot be calculated. The fitting curves based on the above data are shown in FIGS. 10 and 11.

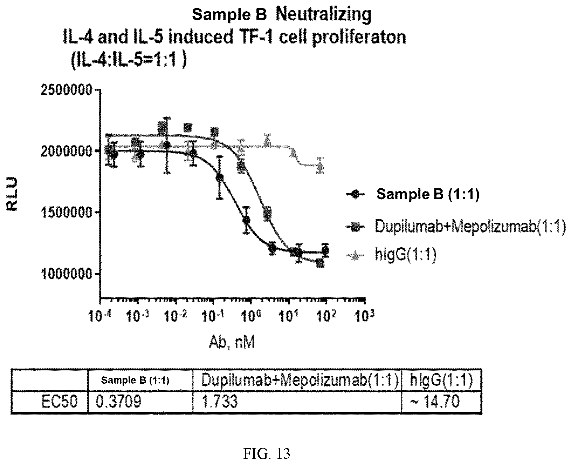

When IL4 : IL5 = 1 : 1, in plate 7, the test window of positive control Dupilumab was 1.77 times, and the EC50 was 0.5701nM; the test window of Mepolizumab was 1.11 times, and the EC50 was 14.89 nM; the test window of sample B bivalent antibody was 1.71 times, and the EC50 was 0.3692 nM; in plate 8, the window of sample B bivalent antibody was 1.70 times, and the EC50 was 0.3709 nM; the window of positive control Dupilumab + Mepolizumab was 1.92 times, and the EC50 was about 1.733 nM; the window of negative control human IgG was 1.07 times, and the EC50 was approximately 14.70 nM. The fitting curves based on the above data are shown in FIGS. 12 and 13.

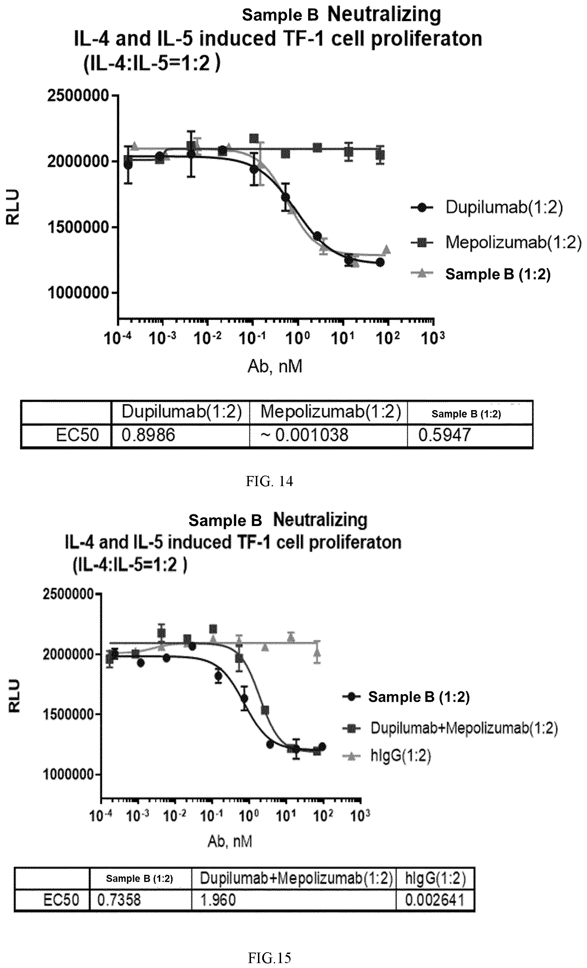

When IL4 : IL5 = 1 : 2, in plate 9, the test window of positive control Dupilumab was 1.68 times, and the EC50 was 0.8986 nM; the test window of Mepolizumab was 1.06 times, and the EC50 was 0.001038 nM; the test window of Sample B bivalent antibody was 1.58 times, and the EC50 was 0.5947 nM; in plate 10, the window of the sample B bivalent antibody was 1.64 times, and the EC50 was 0.7358 nM; the window of the positive control Dupilumab + Mepolizumab was 1.62 times, and the EC50 was about 1.960 nM; the window of negative control human IgG was 0.99 times, and the EC50 was approximately 0.002641 nM. The fitting curves based on the above data are shown in FIGS. 14 and 15.

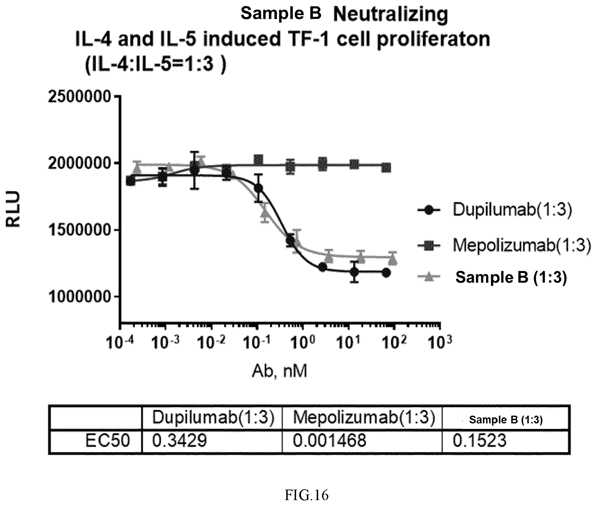

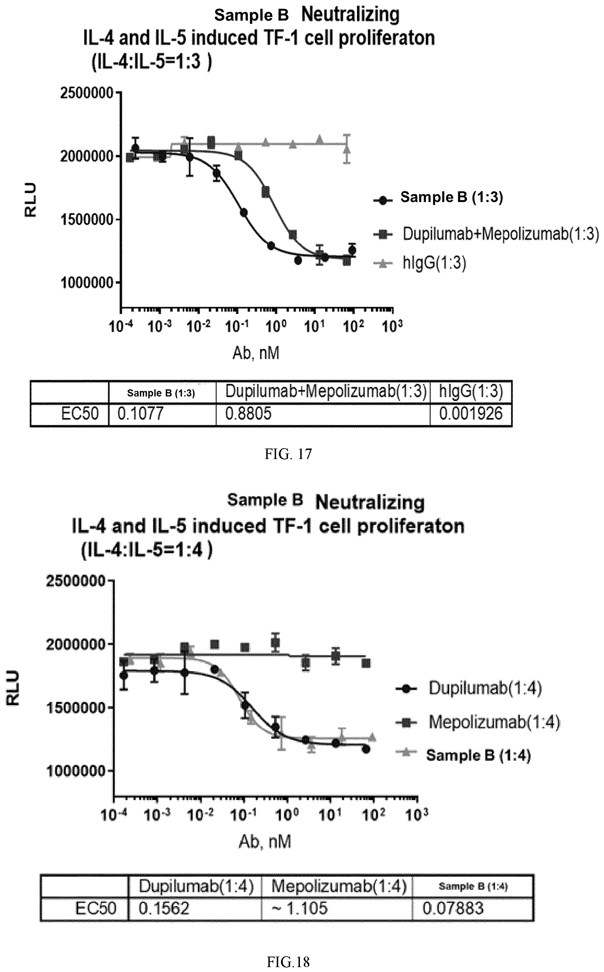

When IL4 : IL5 = 1 : 3, in plate 11, the test window of positive control Dupilumab was 1.53 times, and the EC50 was 0.3429 nM; the test window of Mepolizumab was 1.01 times, and the EC50 was 0.001468 nM; the test window of sample B bivalent antibody was 1.50 times, and the EC50 was 0.1523 nM; in plate 12, the window of sample B bivalent antibody was 1.74 times, and the EC50 was 0.1077 nM; the window of positive control Dupilumab + Mepolizumab was 1.76 times, and the EC50 was about 0.8805 nM; the window of negative control human IgG was 0.95 times, and the EC50 was approximately 0.001926 nM. The fitting curves based on the above data are shown in FIGS. 16 and 17.

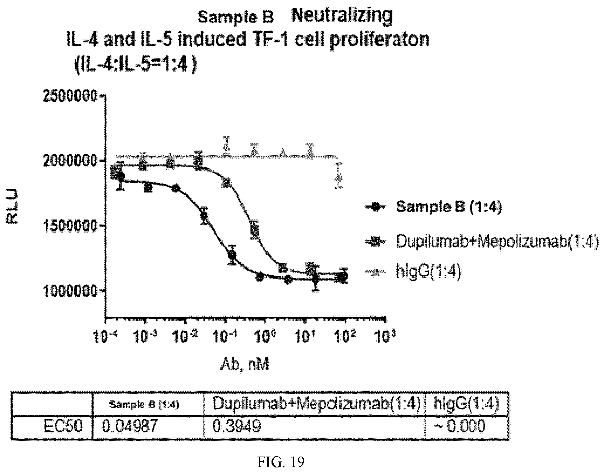

When IL4 : IL5 = 1 : 4, in plate 13, the test window of positive control Dupilumab was 1.58 times, and the EC50 was 0.1562 nM; the test window of Mepolizumab was 1.14 times, and the EC50 was 1.105 nM; the test window of sample B bivalent antibody was 1.50 times, and the EC50 was 0.07883 nM; in plate 14, the window of the sample B bivalent antibody was 1.67 times, and the EC50 was 0.04987 nM; the window of the positive control Dupilumab + Mepolizumab was 1.79 times, and the EC50 was about 0.3949 nM; the window of negative control human IgG was 1.03 times and the EC50 was approximately 0. The fitting curves based on the above data are shown in FIGS. 18 and 19.

Conclusion: under the above assay conditions, in the assay for mixed IL-4 and IL-5 at different ratios, the window of the bivalent antibody sample B was comparable to those of Dupilumab and mixed Dupilumab + Mepolizumab, respectively, and the EC50 was significantly lower than that of Dupilumab and mixed Dupilumab + Mepolizumab, respectively. Therefore, the neutralizing activity of bivalent antibody sample B was similar to Dupilumab and better than mixed Dupilumab + Mepolizumab.

Example 11

Formulation

1. The formulation, which was an aqueous solution, of the bispecific antibody (4E9V10-2B3V2-2) targeting IL-4R$\alpha$ and IL-5 prepared in Example 4 was formulated as follows:

1.1 Tween-free buffer (10 mM acetate-sodium acetate, 120 mM sucrose, 75 mM glycine, 25 mM arginine hydrochloride, pH 5.0) was formulated as follows.

1) Sodium acetate, sucrose, glycine, and arginine hydrochloride were accurately weighed according to Table 13, dissolved in about 800 ml of ultrapure water, and mixed wells.

2) A 50 ml beaker was prepared, acetic acid was weighed according to Table 13 and rinsed repeatedly with ultrapure water, and all acetic acid was transferred to the solution obtained in the above step 1), and mixed well.

3) The solution was transferred to a 1 L volumetric flask and accurately made up to the volume, and the volumetric flask was inverted repeatedly to mix well.

Table 13 Formula of formulation buffer

| Excipients | Producer | Lot No. | Weight (g/L) |
|---|---|---|---|
| Acetic acid | Merck | 1.00056.2500 | 0.196 |
| Sodium acetate | Merck | 1.37012.5000 | 0.918 |
| Sucrose | Pfanstiehl | S-124-2-MC | 41.075 |
| Glycine | Merck | 1.03669.1000 | 5.630 |
| Arginine hydrochloride | Shanghai Ajinomoto | N/A | 5.267 |

1.2 High-concentration Tween stock buffer (10 mM acetic acid-sodium acetate, 120 mM sucrose, 75 mM glycine, 25 mM arginine hydrochloride, 5% polysorbate 80, pH 5.0) was formulated as follows. Sodium acetate is sodium acetate trihydrate.

a) Sodium acetate, sucrose, glycine, and arginine hydrochloride were accurately weighed according to Table 14, dissolved in about 100 ml of ultrapure water, and mixed wells.
b) A 50 ml beaker was prepared, acetic acid was weighed according to Table 14 and rinsed repeatedly with ultrapure water, and all acetic acid was transferred to the solution obtained in the above step a), and mixed well.
c) two 50 ml centrifuge tubes were prepared, polysorbate 80 was accurately weighed according to Table 14, 35 ml of the solution obtained in the above step b) was used, polysorbate 80 was dissolved by vortex, mixed well, and rinsed repeatedly with the solution obtained in the above step b), and all polysorbate 80 was transferred to the solution obtained in the above step b) and mixed well.

3) The solution was transferred to a 250 ml volumetric flask and accurately made up to the volume, and the volumetric flask was inverted repeatedly to mix well.

Table 14 Formula of Formulation buffer

| Excipients | Producer | Lot No. | Weight (g/250ml) |
|---|---|---|---|
| Acetic acid | Merck | 1.00056.2500 | 0.049 |
| Sodium acetate | Merck | 1.37012.5000 | 0.230 |
| Sucrose | Pfanstiehl | S-124-2-MC | 10.269 |
| Glycine | Merck | 1.03669.1000 | 1.408 |
| Arginine hydrochloride | Shanghai Ajinomoto | N/A | 1.317 |
| Polysorbate 80 | Croda | SR40925/.250/4A2/4 | 12.500 |

1.3 The formulation buffer (10 mM acetate-sodium acetate, 120 mM sucrose, 75 mM glycine, 25 mM arginine hydrochloride, 0.05% polysorbate 80, pH 5.0) was formulated.

The steps were as follows: 1 L of the Tween-free buffer prepared in 1.1 and 10.101 ml of the high-concentration Tween stock buffer prepared in 1.2 were mixed well.

Note: Each buffer can be enlarged or reduced in formulated volume as needed.

To prepare the desired formulation, the antibody protein can be added to the formulation buffer to obtain a formulation with a desired concentration.

2. The assay methods are as follows:

2.1 pH measurement

The measurement was carried out in accordance with General Chapter 0631 "Measurement of pH Value" of the Pharmacopoeia of the People's Republic of China (2015 Edition, Part IV).

2.2 Protein concentration measurement

The measurement was performed using UV spectrophotometry. Ultrapure water was weighed to dilute the sample to be tested, and the absorbance of the diluted sample solution was measured at 280 nm. Two solutions in parallel were prepared for each sample to be tested. The protein content is calculated according to the formula: C (mg/ml) = A $\times$ D / $\varepsilon$, where A is the absorbance value of the solution, D is the dilution factor, and $\varepsilon$ is the extinction coefficient: 1.666 (mg/ml)$^{-1}$ + cm$^{-1}$. The protein content was expressed as an average of two parallel calculations.

2.3 Osmotic pressure (molar concentration assay)

The measurement was carried out in accordance with General Chapter 0632 "Molar concentration assay for osmotic pressure" of the Pharmacopoeia of the People's Republic of China (2015 Edition, Part IV).

2.4 Viscosity

The viscosity of protein was measured using RheoSense micro VISC viscometer.

2.5 Purity (SEC-HPLC)

The measurement was performed using high performance liquid chromatography. Separation was carried out in a size exclusion chromatography column using 50 mmol/L phosphate buffer+300 mmol/L NaCl + 10% acetonitrile, pH 6.8 (7.8 g of NaH$_2$PO$_4$·2H$_2$O and 17.53 g of NaCl were weighed and dissolved in 800 ml of ultrapure water, mixed well, added with 100 ml of acetonitrile, adjusted to pH 6.8 using NaOH, made up to a volume of 1000 ml, filtered through a 0.22 $\mu$m filter membrane, and stored at room temperature) as a mobile phase, with a flow rate of 0.5 ml/min, a detection wavelength of 280 nm, at column temperature of 25 °C. The sample to be tested was diluted with ultrapure water to 2 mg/ml as the sample solution to be tested; the working reference was diluted with ultrapure water to 2 mg/ml as the system applicability solution. The sample buffer was diluted in the same fold with ultrapure water and the diluted sample buffer was used as a blank solution. 50 $\mu$l each of the blank solution, system applicability solution, and sample solution to be tested were used and injected into the liquid chromatograph.

2.6 Purity (nrCE-SDS)

The measurement was performed using capillary gel electrophoresis. Before electrophoresis, the capillary column was rinsed with 0.1 mol/L sodium hydroxide, 0.1 mol/L hydrochloric acid, ultrapure water, and electrophoresis gel 70 psi, respectively. An appropriate amount of the sample to be tested (containing about 100 $\mu$g protein) was weighed, added with 93 $\mu$l of sample buffer, pH 6.0 (0.44 g Na$_2$HPO$_4$·12H$_2$O and 1.36 g NaH$_2$PO$_4$·2H$_2$O were weighed into a 50 ml centrifuge tube, and 90 ml ultrapure water was added for full dissolution and well mixed, followed by ultrapure water to make up the volume to 100 ml to prepare a phosphate buffer solution. 200 $\mu$l of phosphate buffer solution was precisely measured, and added with 100 $\mu$l of 10% sodium dodecyl sulfate solution and 700 $\mu$l of ultrapure water were added and mixed well to obtain the sample buffer), 2 $\mu$l Internal Standard (10 kDa), and 5 $\mu$l 250 mmol/L NEM (31 mg of N-ethylmaleimide was weighed and dissolved in 1 ml of ultrapure water), mixed well, heated at 70 °C for 10 minutes, cooled to room temperature and then transferred to a sample vial as the sample solution to be tested. The sample buffer at same volume as the test sample was used and subjected to the same operation to prepare a blank solution. Sample injection conditions: 5 kV, 20 seconds; separation voltage: 15 kV, 35 minutes. The capillary column temperature was controlled at 25 °C, and the test window width was 100 $\times$ 800 $\mu$m.

2.7 Charge variant (icIEF)

The measurement was performed using imaging capillary isoelectric focusing electrophoresis (iCIEF). The capillary has an inner diameter of 100 $\mu$m and a total length of 5 cm. Before electrophoresis of the sample, the capillary column needed to be rinsed with 0.5% MC (methylcellulose) solution and ultrapure water. The sample was loaded in a vacuum injection manner for 55 seconds. The prefocusing voltage was 1.5 KV for 1 minute, and the focusing voltage was 3 KV for 7 minutes. The sample disk temperature was 10°C, the capillary column temperature was room temperature, and the detection wavelength was 280 nm. 500 mmol/L arginine solution was used as cathode stabilizer, 10 mol/L urea solution was used to improve protein solubility, and 0.5% MC solution was used to reduce the adhesion between protein and capillary. The sample to be tested, after being concentrated and desalted, was diluted with ultrapure water to 1.0 mg/ml. 20 $\mu$l of diluted (1.0 mg/ml) sample, 35 $\mu$l of 1% MC solution, 4 $\mu$l of amphoteric electrolyte (pH 3 to 10), 2 $\mu$l of cathode stabilizer, 3 $\mu$l of 10 mol/L urea solution, 34 $\mu$l of ultrapure water, 1 $\mu$l of pI 6.82 Maker and 1 $\mu$l of pI 9.91 Maker were mixed to prepare a sample solution to be tested. A blank solution and a reference solution were prepared in the same manner using the sample buffer and working reference solution, respectively.

2.8 Binding activity (ELISA method)

400 $\mu$g/ml IL-5 antigen was diluted to 0.5 $\mu$g/ml in 1$\times$ ELISA coating solution, and coated on a 96-well plate at 50.0 $\mu$l/well, and left standing overnight at 2 to 8 °C. The coated ELISA plate was taken out, with the coating solution discarded. ELISA washing solution was added at 250 $\mu$l/well, the plate was left standing for 30 s, and then the ELISA washing solution was discarded. Washing was repeated 4 times. ELISA working solution was added at 250 $\mu$l/well for blocking and the plate was incubated at room temperature for 2 h. After the incubation, the gradient diluted sample was transferred to a 96 well plate at 50.0 $\mu$l/well in duplicate, and incubated at room temperature for 1 h. The 0.5 ug/ml diluted biotinylated human IL-4R was transferred to a 96 well plate at 50.0 $\mu$l/well, and incubated at room temperature for 1 h. The secondary antibody working solution was obtained by 1:10000 diluting streptavidin HRP in ELISA working solution. The secondary antibody working solution was added at 50.0 $\mu$l/well and incubated at room temperature for 1 h. The reaction solution in the 96 well plate that has been reacted with the secondary antibody working solution was discarded. ELISA wash solution was added at 250 $\mu$l/ well and then discarded. Washing was repeated 4 times. The plate was dried by tapping on a clean absorbent paper. TMB chromogenic solution was added at 100 $\mu$l/well and left standing for 10 min at room temperature. 2 mol/l $H_2SO_4$ was added at 50 $\mu$l/well in the 96 well microplate after color development to stop the color reaction. The absorbance value was read at 450 nm on the microplate reader, with 630 nm as the reference wavelength. A four-parameter curve was fitted using Graphpad Prism with the concentration as the abscissa, $OD_{450\text{-}630\ nm}$ as the ordinate, so that $EC_{50}$ and $R^2$ can be directly obtained. The relative binding activity of the sample to be tested can be calculated according to the following formula (the binding activity results were rounded).

$$\text{Relative binding activity (\%)} = \frac{\text{EC50 of Reference (or working reference)}}{\text{EC50 of test sample}} \times 100\%$$

3. Optimization of Excipient Ratio

3.1 Formula Design

A total of 6 groups of formulas were designed, see Table 15.

Table 15 List of formula information

| No. | Composition of formula |
|---|---|
| D01 | 20 mmol/L acetic acid-sodium acetate, 50 mmol/L glycine, 50mmol/L arginine hydrochloride, 100 mmol/L sucrose, 0.05% PS80, pH5.0 |
| D02 | 20 mmol/L acetic acid-sodium acetate, 50 mmol/L glycine, 50 mmol/L arginine hydrochloride, 100 mmol/L sucrose, 10 mmol/L methionine, 0.05% PS80, pH5.0 |
| D03 | 10 mmol/L acetic acid-sodium acetate, 50 mmol/L glycine, 50 mmol/L arginine hydrochloride, 120 mmol/L sucrose, 0.05% PS80, pH5.0 |
| D04 | 10 mmol/L acetic acid-sodium acetate, 75 mmol/L glycine, 25 mmol/L arginine hydrochloride, 120 mmol/L sucrose, 0.05% PS80, pH5.0 |
| D05 | 10 mmol/L acetic acid-sodium acetate, 75 mmol/L glycine, 180 mmol/L sucrose, 0.05% PS80, pH5.0 |
| D06 | 20 mmol/L acetic acid-sodium acetate, 100 mmol/L arginine hydrochloride, 80 mmol/L sucrose, 0.02% PS80, pH5.0 |

The protein 4E9V10-2B3V2-2 was concentrated and subjected to buffer exchange into each formula. After the buffer exchange was completed, the concentration was adjusted so that the target (4E9V10-2B3V2-2) had a concentration of about 100 mg/ml (the pH of each formulation upon addition of 4E9V10-2B3V2-2 was shown in the column T0 in Table 18). After the buffer exchange was completed, except for the samples for freeze-thaw inspection which were placed in the 5ml Flexboy storage bag, the rest were placed in an injection vial made of 2R medium borosilicate glass tube and inspected according to Table 16.

Table 16 Experimental scheme

| Test items | Inspection conditions | Time point | | | | | |
|---|---|---|---|---|---|---|---|
| | | T0 | 48h | 5cycles | 7d | 14d | 30d |
| X: Appearance, pH/protein concentration/SEC-HPLC/nrCE-SDS/iclEF Y: viscosity/osmotic pressure | 5 °C | X, Y | - | - | - | - | X |
| | 25 °C | - | - | - | X | X | - |
| | -20 °C | - | - | - | - | X | X |
| | Freeze/Thaw (-80°C/room temperature) | - | - | X | - | - | - |
| | Shaking (650 rpm) | - | X | - | - | - | - |
| Note: -: there is no such inspection point; T0 is Time Initial (at zero point or at beginning) | | | | | | | |

3.2 Experimental results

It should be noted that in the following results, Agt represents agitation (650 rpm). Ft-5cyc represents five freeze-thaw cycles (-80 °C/room temperature).

(1) Appearance inspection results

Because D05 had obvious stratification during the buffer exchange using ultrafiltration centrifuge tubes, this group of formulas was discarded for investigation. The appearance results of other formulas are shown in Table 17. After 5 freeze-thaw cycles, the samples in all formulas had clear and colorless appearance.

Because the sample was laid flat inside the bag, the slightly yellow color of the sample itself was attenuated.

The formula D01, D02, and D03 samples were all opalescent and slightly yellow at the point T0 and at various temperatures; the formula D04 sample was clear and slightly yellow at the point T0, and was slightly opalescent and slightly yellow at the various temperatures; the formula D06 sample was strong opalescent and slightly yellow at point T0 and at various temperatures.

Table 17 Summary of appearance inspection results

| No. | Zero point (T0) | -20 °C | | 5 °C | 25 °C | | Agt48h | Ft-5cyc |
|---|---|---|---|---|---|---|---|---|
| | | 14d | 30d | 30d | 7d | 14d | | |
| D01 | +++ | +++ | +++ | +++ | +++ | +++ | +++ | 0 |
| D02 | +++ | +++ | +++ | +++ | +++ | +++ | +++ | 0 |
| D03 | +++ | +++ | +++ | +++ | +++ | +++ | +++ | 0 |
| D04 | + | ++ | ++ | ++ | ++ | ++ | ++ | 0 |
| D06 | ++++ | ++++ | ++++ | ++++ | ++++ | ++++ | ++++ | 0 |
| Note: 0: clear and colorless (because of its small amount, the sample was observed directly in the cryopreservation bag without taking out and placing in a penicillin vial); +: clear and slightly yellow; ++: slightly opalescent and slightly yellow; +++: opalescent and slightly yellow; ++++: strong opalescent and slightly yellow. | | | | | | | | |

(2) pH test results

The pH results are shown in Table 18. Compared with T0, there was no significant difference in the pH of the samples in each group of formulas at each inspection point (all within the acceptable range of $\pm 0.3$).

Table 18 Summary of pH results

| No. | pH | | | | | | | |
|---|---|---|---|---|---|---|---|---|
| | T0 | -20 °C | | 5 °C | 25°C | | Agt48h | FT-5cyc |
| | | 14d | 30d | 30d | 7d | 14d | | |
| D01 | 5.2 | 5.1 | 5.2 | 5.1 | 5.1 | 5.2 | 5.2 | 5.1 |

(continued)

| No. | pH | | | | | | Agt48h | FT-5cyc |
|---|---|---|---|---|---|---|---|---|
| | T0 | -20 °C | | 5 °C | 25°C | | | |
| | | 14d | 30d | 30d | 7d | 14d | | |
| D02 | 5.1 | 5.1 | 5.1 | 5.1 | 5.1 | 5.2 | 5.1 | 5.1 |
| D03 | 5.1 | 5.2 | 5.2 | 5.1 | 5.1 | 5.2 | 5.1 | 5.1 |
| D04 | 5.2 | 5.2 | 5.2 | 5.2 | 5.2 | 5.2 | 5.2 | 5.1 |
| D06 | 5.1 | 5.1 | 5.1 | 5.1 | 5.1 | 5.1 | 5.1 | 5.0 |

(3) Protein concentration measurement results

The protein concentration results are shown in Table 19. Compared with the point T0, there was no significant change in the protein concentration of the samples in each group (all within the acceptable range of ±10%).

Table 19 Summary of protein concentration results

| No. | pH | | | | | | Agt48h | FT-5cyc |
|---|---|---|---|---|---|---|---|---|
| | T0 | -20 °C | | 5 °C | 25 °C | | | |
| | | 14d | 30d | 30d | 7d | 14d | | |
| D01 | 107.5 | 106.9 | 100.6 | 105.0 | 108.5 | 109.0 | 108.4 | 106.7 |
| D02 | 104.2 | 106.7 | 108.6 | 103.1 | 106.9 | 107.4 | 107.0 | 106.4 |
| D03 | 105.4 | 107.1 | 103.5 | 105.0 | 107.4 | 106.4 | 105.7 | 100.2 |
| D04 | 108.0 | 106.8 | 107.2 | 105.4 | 107.7 | 107.7 | 108.7 | 108.1 |
| D06 | 109.5 | 108.2 | 106.0 | 115.6 | 114.8 | 108.9 | 108.4 | 103.6 |

The viscosity and osmotic pressure results are shown in Table 20. The viscosity in all formulas was within the range of 5.7m to 6.1mPa.s, and the osmotic pressure was within the range of 299 mOsmol/kg to 331 mOsmol/kg.

Table 20 Summary of viscosity and osmotic pressure results

| No. | Viscosity (mPa.s) | osmotic pressure (mOsmol/kg) |
|---|---|---|
| D01 | 6.032 | 313.5 |
| D02 | 5.713 | 319.5 |
| D03 | 6.066 | 317.5 |
| D04 | 6.057 | 299.5 |
| D06 | 5.854 | 330.5 |

(5) Purity (SEC-HPLC) test results

The results of SEC-HPLC (Size Exclusion-High Performance Liquid Chromatography) are shown in Table 21 to Table 23. Compared with T0, after 14 days of inspection at 25 °C and 48 h of shaking, the monomer content decreased, however, by within 1.0%. Under the conditions of -20 °C, 5°C or freeze-thaw inspection, there was basically no difference in the monomer content of the samples in all formulas. Compared with T0, the monomer content varied within 0.2%. From the purity results (from SEC-HPLC), there was no significant difference between the components.
Table 21 Purity test results (SEC-HPLC_Aggregate)

| No. | Aggregate % | | | | | | | |
|-----|----|------|------|------|------|------|--------|---------|
|     | T0 | -20 °C | | 5 °C | 25 °C | | Agt48h | FT-5cyc |
|     |    | 14d | 30d | 30d | 7d | 14d |        |         |
| D01 | 3.6 | 3.7 | 3.6 | 3.9 | 4.0 | 4.2 | 4.0 | 3.7 |
| D02 | 3.6 | 3.6 | 3.6 | 3.8 | 3.9 | 4.1 | 3.9 | 3.6 |
| D03 | 3.7 | 3.6 | 3.7 | 3.9 | 4.0 | 4.2 | 4.0 | 3.6 |
| D04 | 3.7 | 3.7 | 3.6 | 3.8 | 4.0 | 4.2 | 3.8 | 3.7 |
| D06 | 3.7 | 3.7 | 3.5 | 3.5 | 4.2 | 4.2 | 4.3 | 3.6 |

Table 22 Purity test results (SEC-HPLC monomer)

| No. | Monomer % | | | | | | | |
|-----|----|------|------|------|------|------|--------|---------|
|     | T0 | -20 °C | | 5 °C | 25 °C | | Agt48h | FT-5cyc |
|     |    | 14d | 30d | 30d | 7d | 14d |        |         |
| D01 | 96.3 | 96.3 | 96.3 | 96.1 | 96.0 | 95.7 | 95.9 | 96.3 |
| D02 | 96.3 | 96.3 | 96.4 | 96.2 | 96.0 | 95.9 | 96.1 | 96.3 |
| D03 | 96.3 | 96.3 | 96.3 | 96.1 | 95.9 | 95.7 | 95.9 | 96.3 |
| D04 | 96.3 | 96.3 | 96.4 | 96.2 | 96.0 | 95.8 | 96.2 | 96.3 |
| D06 | 96.3 | 96.3 | 96.5 | 96.5 | 95.8 | 95.8 | 95.6 | 96.3 |

Table 22 Purity test results (SEC-HPLC_fragment)

| No. | Fragment % | | | | | | | |
|-----|----|------|------|------|------|------|--------|---------|
|     | T0 | -20 °C | | 5 °C | 25 °C | | Agt48h | FT-5cyc |
|     |    | 14d | 30d | 30d | 7d | 14d |        |         |
| D01 | 0.1 | 0.1 | 0.0 | 0.0 | 0.1 | 0.1 | 0.1 | 0.1 |
| D02 | 0.1 | 0.1 | 0.1 | 0.0 | 0.1 | 0.0 | 0.1 | 0.1 |
| D03 | 0.1 | 0.1 | 0.1 | 0.1 | 0.1 | 0.1 | 0.1 | 0.1 |
| D04 | 0.1 | 0.1 | 0.0 | 0.0 | 0.1 | 0.1 | 0.1 | 0.1 |
| D06 | 0.0 | 0.1 | 0.1 | 0.1 | 0.1 | 0.0 | 0.1 | 0.1 |

(6) Purity (nrCE-SDS) test results

The results of nrCE-SDS (non-reducing capillary electrophoresis) are shown in Tables 24 to 26. Compared with T0, the main peak content of formula D01 to D04 samples varied less than 1.0% under all inspection conditions. The main peak content of formula D06 sample after shaking inspection was significantly reduced.

Table 24 Purity test results (nrCE-SDS main peak)

| No. | Main peak % | | | | | | | |
|-----|----|------|------|------|------|------|--------|---------|
|     | T0 | -20 °C | | 5 °C | 25 °C | | Agt48h | Ft-5cyc |
|     |    | 14d | 30d | 30d | 7d | 14d |        |         |
| D01 | 99.3 | 99.2 | 99.3 | 99.3 | 98.9 | 98.6 | 99.2 | 99.3 |
| D02 | 99.2 | 99.2 | 99.7 | 99.6 | 99.0 | 98.6 | 99.1 | 99.2 |
| D03 | 99.3 | 99.4 | 99.3 | 99.3 | 98.9 | 99.0 | 99.2 | 99.0 |

(continued)

| No. | Main peak % | | | | | | | |
|---|---|---|---|---|---|---|---|---|
| | T0 | -20 °C | | 5 °C | 25 °C | | Agt48h | Ft-5cyc |
| | | 14d | 30d | 30d | 7d | 14d | | |
| D04 | 99.3 | 99.5 | 99.3 | 99.3 | 99.1 | 98.6 | 98.9 | 99.0 |
| D06 | 99.4 | 99.1 | 99.7 | 99.3 | 98.9 | 98.7 | 89.9 | 99.0 |

Table 25 Purity test results (nrCE-SDS_fragment)

| No. | Fragment % | | | | | | | |
|---|---|---|---|---|---|---|---|---|
| | T0 | -20 °C | | 5 °C | 25 °C | | Agt48h | FT-5cyc |
| | | 14d | 30d | 30d | 7d | 14d | | |
| D01 | 0.0 | 0.0 | 0.0 | 0.0 | 0.0 | 0.0 | 0.0 | 0.0 |
| D02 | 0.0 | 0.0 | 0.0 | 0.0 | 0.0 | 0.0 | 0.0 | 0.0 |
| D03 | 0.0 | 0.0 | 0.0 | 0.0 | 0.0 | 0.0 | 0.0 | 0.0 |
| D04 | 0.0 | 0.0 | 0.0 | 0.0 | 0.0 | 0.0 | 0.0 | 0.0 |
| D06 | 0.0 | 0.0 | 0.0 | 0.0 | 0.0 | 0.0 | 0.0 | 0.0 |

Table 26 Purity test results (nrCE-SDS_Aggregate)

| No. | Aggregate % | | | | | | | |
|---|---|---|---|---|---|---|---|---|
| | T0 | -20 °C | | 5 °C | 25 °C | | Agt48h | FT-5cyc |
| | | 14d | 30d | 30d | 7d | 14d | | |
| D01 | 0.7 | 0.8 | 0.7 | 0.7 | 1.1 | 1.4 | 0.8 | 0.7 |
| D02 | 0.8 | 0.8 | 0.4 | 0.4 | 1.0 | 1.4 | 0.9 | 0.8 |
| D03 | 0.7 | 0.6 | 0.7 | 0.7 | 1.1 | 1.0 | 0.8 | 1.1 |
| D04 | 0.7 | 0.5 | 0.7 | 0.7 | 0.9 | 1.4 | 1.1 | 1.0 |
| D06 | 0.6 | 0.9 | 0.4 | 0.8 | 1.1 | 1.3 | 1.1 | 0.9 |

(7) Charge variant (icIEF) test results

icIEF is whole-column imaging capillary isoelectric focusing electrophoresis. The charge variant (icIEF) results are shown in Tables 27 to 29. Based on the results in tables, it can be seen that the value of the main peak content of the formula D04 sample varied relatively little, so the formula D04 can be used as an alternative formula.

Table 27 Charge variant test results (icIEF_acidic component)

| No. | Acidic component % | | | | | | | |
|---|---|---|---|---|---|---|---|---|
| | T0 | -20 °C | | 5 °C | 25 °C | | Agt48h | FT-5cyc |
| | | 14d | 30d | 30d | 7d | 14d | | |
| D01 | 41.2 | 39.5 | 37.5 | 42.9 | 39.9 | 40.0 | 40.4 | 41.3 |
| D02 | 41.5 | 42.1 | 41.9 | 42.3 | 40.0 | 42.2 | 40.1 | 40.3 |
| D03 | 41.0 | 43.2 | 42.6 | 38.0 | 41.0 | 42.2 | 41.0 | 41.0 |
| D04 | 41.1 | 42.7 | 42.1 | 42.0 | 40.6 | 38.9 | 40.9 | 41.7 |
| D06 | 41.1 | 42.9 | 42.2 | 39.1 | 40.7 | 39.3 | 39.8 | 40.8 |

Table 28 Charge variant test results (icIEF _main peak)

| No. | Main peak % | | | | | | | |
|-----|-----|-----|-----|-----|-----|-----|-----|-----|
| | T0 | -20 °C | | 5 °C | 25 °C | | Agt48h | FT-5cyc |
| | | 14d | 30d | 30d | 7d | 14d | | |
| D01 | 55.4 | 57.9 | 60.8 | 52.5 | 55.3 | 52.7 | 55.6 | 55.7 |
| D02 | 55.2 | 54.3 | 54.9 | 53.2 | 55.2 | 50.2 | 55.8 | 56.4 |
| D03 | 55.7 | 52.8 | 53.9 | 59.8 | 54.7 | 50.7 | 54.9 | 55.8 |
| D04 | 55.9 | 53.8 | 54.7 | 54.1 | 55.1 | 55.4 | 55.3 | 55.3 |
| D06 | 55.9 | 53.4 | 54.1 | 57.6 | 54.6 | 54.1 | 56.4 | 56.3 |

Table 29 Charge variant test results (icIEF_basic component)

| No. | Basic component % | | | | | | | |
|-----|-----|-----|-----|-----|-----|-----|-----|-----|
| | T0 | -20 °C | | 5 °C | 25 °C | | Agt48h | FT-5cyc |
| | | 14d | 30d | 30d | 7d | 14d | | |
| D01 | 3.3 | 2.5 | 1.7 | 4.6 | 4.8 | 7.3 | 4.0 | 3.0 |
| D02 | 3.3 | 3.7 | 3.2 | 4.5 | 4.8 | 7.6 | 4.0 | 3.3 |
| D03 | 3.3 | 3.9 | 3.5 | 2.2 | 4.3 | 7.2 | 4.1 | 3.1 |
| D04 | 3.0 | 3.4 | 3.2 | 3.9 | 4.3 | 5.7 | 3.8 | 3.1 |
| D06 | 3.1 | 3.7 | 3.7 | 3.3 | 4.7 | 6.5 | 3.8 | 2.9 |

From the purity (SEC-HPLC, nrCE-SDS) results, it can be seen that compared with T0, the monomer content (SEC-HPLC) and main peak content (nrCE-SDS) varied within 1.0%; and from the charge variant (icIEF) results, it can be seen that the main peak content of the formula D04 sample varied relatively little.

Formula E01 (DO4) (10 mmol/L acetic acid-sodium acetate, 75 mmol/L glycine, 25 mmol/L arginine hydrochloride, 120 mmol/L sucrose, 0.05% PS80, pH 5.0, protein concentration 100 mg/ml) was confirmed for its formulation.

The confirmation scheme is shown in Table 30. The protein was concentrated and subjected to buffer exchange into each formula. After the buffer exchange was completed, except for the samples for freeze-thaw inspection which were placed in the 5ml Flexboy storage bag, the rest were placed in an injection vial made of 2R medium borosilicate glass tube and inspected according to Table 30.

Table 30 Experimental scheme for formula confirmation

| Test items | Inspection conditions | Time point | | | | | | |
|-----|-----|-----|-----|-----|-----|-----|-----|-----|
| | | T0 | 7d | 14d | 30d | 91d | 48h | 5cycles |
| X: Appearance, pH/protein concentration/SEC-HPLC/nrCE-SDS/iclEF Y: viscosity/osmotic pressure | 5°C | X,Y, Z | - | X | X,Z | X,Z | - | - |
| | 37°C | - | X | X | - | - | - | - |
| | -20°C | - | - | X | X,Z | X,Z | - | - |
| | Freezing/Th awing(-80°C/RT) | - | - | - | - | - | - | X |
| | Shaking (650rpm) | - | - | - | - | - | X | - |
| Note: -: there is no such inspection point | | | | | | | | |

Experimental results

(1) Viscosity and osmotic pressure test results

The viscosity and osmotic pressure results are shown in Table 31. The viscosity was 4.590 mPa.s. The osmotic pressure was 286 mOsmol/kg.

Table 31 Viscosity and osmotic pressure results in formula confirmation

| No. | Viscosity (mPa.s) | Osmotic pressure (mOsmol/kg) |
|---|---|---|
| E01 | 4.590 | 286 |

(2) Appearance, pH, and protein concentration test results

The appearance, pH, and protein concentration results are shown in Table 32. Under each inspection condition, the appearance was clear and yellow. Due to the difference in sample sources, the appearance of formula during confirmation was better than the sample status of the formula D04 during screening. There was no significant difference in pH (within the acceptable range of ±0.3) and in protein concentration (within the acceptable range of ±10%) under various inspection conditions.

Table 32 Results of appearance, pH, and protein concentration in formula confirmation

| No. | T0 | -20 °C | | | 5 °C | | | 37 °C | | Agt 48h | FT-5cyc |
|---|---|---|---|---|---|---|---|---|---|---|---|
| | | 14d | 30d | 91d | 14d | 30d | 91d | 7d | 14d | | |
| Appearance | + | + | + | + | + | + | + | + | + | + | + |
| pH | 5.2 | 5.1 | 5.1 | 5.1 | 5.1 | 5.1 | 5.2 | 5.2 | 5.1 | 5.2 | 5.1 |
| Protein concentration (mg/ml) | 97.2 | 98.2 | 97.2 | 98.3 | 98.3 | 97.5 | 98.6 | 95.5 | 97.4 | 98.5 | 96.2 |
| Note: +: clear and yellow | | | | | | | | | | | |

(4) Purity (SEC-HPLC) test results

The purity (SEC-HPLC) test results are shown in Table 33. After 14 days of inspection at 37°C, the monomer content decreased by 2.6%. Under conditions of -20 °C, 5 °C, shaking, or freezing and thawing, the content of monomer in the sample varied by no more than 1.5%.

Table 33 Purity test result in formula confirmation (SEC-HPLC)

| Components | T0 | -20 °C | | | 5 °C | | | 37 °C | | Agt 48h | FT-5cyc |
|---|---|---|---|---|---|---|---|---|---|---|---|
| | | 14d | 30d | 91d | 14d | 30d | 91d | 7d | 14d | | |
| Aggregate% | 2.6 | 2.7 | 2.7 | 2.8 | 2.9 | 3.1 | 3.7 | 4.4 | 5.2 | 2.8 | 2.6 |
| Monomer% | 97.3 | 97.2 | 97.2 | 97.1 | 97.0 | 96.8 | 96.2 | 95.5 | 94.7 | 97.1 | 97.3 |
| Fragment% | 0.1 | 0.1 | 0.1 | 0.1 | 0.1 | 0.1 | 0.1 | 0.1 | 0.1 | 0.1 | 0.1 |

(4) Purity (nrCE-SDS) test results

The purity (nrCE-SDS) test results are shown in Table 34. After 14 days of inspection at 37°C, the monomer content decreased by 1.9%. Under conditions of -20 °C, 5 °C, shaking, or freezing and thawing, the content of monomer in the sample varied by no more than 1.0%.

Table 34 purity test results in formula confirmation (nrCE-SDS)

| Components | T0 | -20 °C | | | 5 °C | | | 37 °C | | Agt 48h | FT-5cyc |
|---|---|---|---|---|---|---|---|---|---|---|---|
| | | 14d | 30d | 91d | 14d | 30d | 91d | 7d | 14d | | |
| Monomer% | 99.8 | 99.8 | 99.6 | 99.2 | 99.4 | 99.5 | 99.3 | 99.3 | 97.9 | 99.6 | 99.5 |
| Fragment% | 0.0 | 0.0 | 0.0 | 0.0 | 0.0 | 0.0 | 0.0 | 0.0 | 0.0 | 0.0 | 0.0 |
| Aggregate% | 0.2 | 0.2 | 0.5 | 0.8 | 0.6 | 0.5 | 0.7 | 0.7 | 2.1 | 0.5 | 0.5 |

(5) Charge variant (icIEF) test results

The test results of the charge variant (icIEF) are shown in Table 35. After 14 days of investigation at 37 °C, compared with T0, the content of the main peak decreased by 5.2%, indicating that the contents of each components of the charge variants in the sample varied under high temperature conditions. Under conditions of -20 °C, 5 °C, shaking, or freezing and thawing, the main peak content in the sample varied by no more than 2.0%.

Table 35 Charge variant test results in formula confirmation (icIEF)

| Components | T0 | -20 °C | | | 5 °C | | | 37 °C | | Agt 48h | FT-5cyc |
|---|---|---|---|---|---|---|---|---|---|---|---|
| | | 14d | 30d | 91d | 14d | 30d | 91d | 7d | 14d | | |
| Acidic component % | 49.3 | 47.6 | 47.4 | 47.6 | 48.1 | 47.4 | 46.5 | 47.2 | 47.4 | 48.6 | 48.4 |
| Main peak% | 48.4 | 49.7 | 49.8 | 50.3 | 49.2 | 49.7 | 50.1 | 44.8 | 43.2 | 48.0 | 49.0 |
| Basic component % | 2.4 | 2.6 | 2.8 | 2.0 | 2.7 | 2.8 | 3.4 | 8.0 | 9.5 | 3.4 | 2.5 |

(6) Binding activity test results

The binding activity (ELISA) test results are shown in Table 36. After the 4E9V10-2B3V2-2 protein was tested at -20 °C and 5 °C for 30 days and 91 days, the binding activity (ELISA) results were all qualified judgment standard: 70% ~130%).

Table 36 Summary of binding activity test results

| | T0 | -20 °C | | 5 °C | |
|---|---|---|---|---|---|
| | | 30d | 91d | 30d | 91d |
| Binding activity % | 92 | 77 | 80 | 78 | 80 |

(7) Summary

From all the results in formula confirmation, it can be seen that under the conditions of -20 °C, 5 °C, shaking, or freeze-thaw inspection, the respective components varied little, and after 91 days of inspection at -20 °C and 5 °C, the binding activity was qualified, indicating that, 4E9V10-2B3V2-2 in the formulation containing formula E01 (D04) has good stability under the conditions of 5 °C, -20 °C, shaking, or freezing and thawing.

Although the embodiments of the present disclosure have been disclosed above, they are not limited to the applications listed in the description and embodiments. They can be applied to various fields suitable for the present disclosure. Additional modifications may be readily implemented by those skilled in the art, and the disclosure is therefore not limited to the specific details without departing from the general concept defined by the claims and equivalent scope.

**Claims**

1. A stable antibody formulation comprising a therapeutically effective amount of an antibody molecule, a tonicity agent, a buffer agent, and a surfactant, wherein the formulation has a pH of 4.5 to 5.5; the antibody molecule is capable of specifically binding to both IL-5 and IL-4R$\alpha$, and comprises at least two immunoglobulin variable domains comprising a first immunoglobulin variable domain capable of specifically binding to IL-4R$\alpha$ molecule and a second immunoglobulin variable domain capable of specifically binding to IL-5.

2. The stable antibody formulation of claim 1, wherein the formulation has a pH of 4.7 to 5.3.

3. The stable antibody formulation of claim 1, wherein the first immunoglobulin variable domain is a first single-domain antibody molecule and comprises CDR1 as set forth in SEQ ID NO: 1, CDR2 as set forth in SEQ ID NO: 2, and CDR3 as set forth in SEQ ID NO: 3; and the second immunoglobulin variable domain is a second single-domain antibody molecule and comprises CDR1 as set forth in SEQ ID NO: 4, CDR2 as set forth in SEQ ID NO: 5, and CDR3 as set forth in SEQ ID NO: 6.

4. The stable antibody formulation of claim 3, wherein the antibody molecule is a polypeptide fusion comprising no linker group between the first single-domain antibody molecule and the second single-domain antibody molecule.

5. The stable antibody formulation of claim 3, wherein the antibody molecule is a polypeptide fusion comprising a biocompatible polymer and the first single-domain antibody molecule and the second single-domain antibody molecule of the polypeptide fusion are ligated via a biocompatible polymer as a linker group.

6. The stable antibody formulation of claim 3, wherein the first single-domain antibody molecule and the second single-domain antibody molecule are ligated via a human IgG Fc region; the antibody molecule has a homodimer structure, and is named 4E9V10-2B3V2; each single-chain Fc fusion proteins is composed of a first immunoglobulin variable domain, a human IgG Fc region, and a second immunoglobulin variable domain.

7. The stable antibody formulation of claim 6, wherein the human IgG Fc region is derived from human IgG1, IgG2, IgG3 or IgG4.

8. The stable antibody formulation of claim 6, wherein the human IgG Fc region is derived from human IgG4 region; and the Fc region has an amino acid sequence as set forth in SEQ ID NO: 13, or at positions 116 to 356 of SEQ ID NO: 9.

9. The stable antibody formulation of claim 1, wherein the first immunoglobulin variable domain has an amino acid sequence as set forth in SEQ ID NO: 7, and the second immunoglobulin variable domain has an amino acid sequence as set forth in SEQ ID NO: 8.

10. A stable antibody formulation, comprising a therapeutically effective amount of an antibody molecule, a tonicity agent, a buffer agent, and a surfactant, wherein the formulation has a pH of 4.5 to 5.5; and the antibody molecule comprises a fusion protein having an amino acid sequence as set forth in SEQ ID NO: 9 or 10.

11. The stable antibody formulation of claim 1 or 10, wherein the buffer agent is an acetic acid-sodium acetate buffer, the tonicity agent comprises sucrose, and the surfactant comprises polysorbate.

12. The stable antibody formulation of claim 11, further comprising a filler comprising glycine, and arginine hydrochloride.

13. The stable antibody formulation of claim 11, wherein acetic acid-sodium acetate has a concentration of 10 to 20 mmol/L, and/or arginine hydrochloride has a concentration of 25 to 100 mmol/L, and/or sucrose has a concentration of 80 to 120 mmol/L, and/or glycine has a concentration of 50 to 75 mmol/L, and/or polysorbate 80 has a concentration of 0.02 to 0.05 w/v%.

14. The stable antibody formulation of claim 1, further comprising methionine.

15. The stable antibody formulation of claim 14, further comprising methionine at a concentration of 10 mmol/L.

16. The stable antibody formulation of claim 1, wherein the antibody molecule is present in an amount of less than 120 mg/mL.

17. The stable antibody formulation of claim 1, wherein the antibody molecule has a concentration of about 0.01 to about 100 mg/ml.

18. The stable antibody formulation of claim 1, comprising 100 mg/mL anti-IL-4 R$\alpha$ /IL-5 bispecific antibody, 20 mmol/L acetic acid-sodium acetate, 50 mmol/L glycine, 50 mmol/L arginine hydrochloride, 100 mmol/L sucrose and 0.05 w/v% of polysorbate 80, with pH of 5.0 to 5.2.

19. The stable antibody formulation of claim 2, comprising 100 mg/mL anti-IL-4 R$\alpha$ /IL-5 bispecific antibody, 20 mmol/L acetic acid-sodium acetate, 50 mmol/L glycine, 50 mmol/L arginine hydrochloride, 100 mmol/L sucrose, 10 mmol/L methionine and 0.05 w/v% of polysorbate 80, with pH of 5.0 to 5.2.

20. The stable antibody formulation of claim 1, comprising 100 mg/mL anti-IL-4 R$\alpha$ /IL-5 bispecific antibody, 10 mmol/L acetic acid-sodium acetate, 50 mmol/L glycine, 50 mmol/L arginine hydrochloride, 120 mmol/L sucrose and 0.05 w/v% of polysorbate 80, with pH of 5.0 to 5.2.

21. The stable antibody formulation of claim 1, comprising 100 mg/mL anti-IL-4 R$\alpha$ /IL-5 bispecific antibody, 10 mmol/L acetic acid-sodium acetate, 75 mmol/L glycine, 25 mmol/L arginine hydrochloride, 120 mmol/L sucrose and 0.05 w/v% of polysorbate 80, with pH of 5.0 to 5.2.

22. The stable antibody formulation of claim 1, comprising 100 mg/mL anti-IL-4 R$\alpha$ /IL-5 bispecific antibody, 20 mmol/L acetic acid-sodium acetate, 100 mmol/L arginine hydrochloride, 80 mmol/L sucrose and 0.02 w/v% of polysorbate 80, with pH of 5.0 to 5.2.

23. The stable antibody formulation of claim 1, further comprising one or more selected from preservatives and/or excipients.

24. The stable antibody formulation of claim 1, wherein a content of monomer in the formulation decreases by no more than 5% after storage at about 37 °C for 14 days.

25. The stable antibody formulation of claim 1, under conditions of -20 °C, 5 °C, shaking, or freezing and thawing, a content of monomer in the sample changes by no more than 1.5%.

26. A container comprising the stable antibody formulation of any one of claims 1-25.

27. The container of claim 26, wherein the container comprises a syringe or bottle made of any one or more of glass, plastic, or polymeric materials.

28. A sealed package comprising the stable antibody formulation of any one of claims 1-25 or a container of claim 26.

29. A kit, comprising the antibody formulation of any one of claims 1-25 and a container containing the antibody formulation.

30. The antibody formulation according to any one of claims 1-25 or the kit of claim 29, for use in preparing a medicament for preventing and/or treating an autoimmune disease.

31. Use of the stable antibody formulation of any one of claims 1-25 in preparing a medicament for treating a disease, wherein the disease comprises asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

32. The use of claim 31, wherein the medicament is a medicament administered by an intravenous or subcutaneous route.

33. A method for treating a disease using the stable antibody formulation of any one of claims 1-25, wherein the disease comprises asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

34. A delivery system comprising the stable antibody formulation of any one of claims 1-25, wherein the delivery system is selected from a disposable syringe.

35. A method for preparing the stable antibody formulation of any one of claims 1-25, comprising: step 1) preparing the antibody molecule; and step 2) mixing a therapeutically effective amount of the antibody molecule with a tonicity agent, a buffer agent, a surfactant and/or other required components to form a stable solution.

36. A medicament for treating a disease, comprising the antibody formulation of any one of claims 1-25, wherein the disease comprises asthma, allergic dermatitis, eczema, arthritis, herpes, chronic primary urticaria, scleroderma, hypertrophic scars, chronic obstructive pulmonary disease, atopic dermatitis, idiopathic pulmonary fibrosis, Kawasaki disease, sickle cell disease, Graves' disease, Sjögren's syndrome, autoimmune lymphoproliferative syndrome, autoimmune hemolytic anemia, Barrett's esophagus, autoimmune uveitis, tuberculosis, and kidney disease, autoimmune diseases, treatment of adults with eosinophilic granulomatosis with polyangiitis, severe eosinophilic asthma, hypereosinophilic syndrome, chronic sinusitis with nasal polyps, chronic obstructive pulmonary disease, uncontrolled chronic sinusitis without nasal polyps, allergic fungal sinusitis, nodular prurigo, familial cold urticaria, bullous pemphigoid, allergic fungal rhinosinusitis, chronic induced cold urticaria, peanut allergy, pollen allergy, eosinophilic esophagitis, keloids, pruritus, allergic bronchopulmonary aspergillosis, seasonal allergic rhinitis.

37. Use of glycine in preparing the stable antibody formulation of any one of claims 1-25, wherein the antibody molecule would still be present stably upon a concentration of the antibody molecule in the antibody formulation reaching 100 mg/mL.

38. Use of the antibody formulation of any one of claims 1-25 in improving the stability of a high- concentration antibody in liquid formulations.

FIG. 1

FIG. 2

FIG. 3

FIG. 4

FIG. 5

**Sample B  Neutralizing**
**IL-4 and IL-5 induced TF-1 cell proliferaton**
**(IL-4:IL-5=4:1 )**

- Dupilumab(4:1)
- Mepolizumab(4:1)
- Sample B (4:1)

| | Dupilumab(4:1) | Mepolizumab(4:1) | Sample B (4:1) |
|---|---|---|---|
| EC50 | 0.8127 | 0.001298 | 0.4245 |

FIG. 6

Sample B **Neutralizing**
**IL-4 and IL-5 induced TF-1 cell proliferaton**
**(IL-4:IL-5=4:1 )**

|  | Sample B (4:1) | Dupilumab+Mepolizumab(4:1) | hIgG(4:1) |
|---|---|---|---|
| EC50 | 0.3212 | 0.8250 | ~ 0.1051 |

FIG. 7

Sample B **Neutralizing**
**IL-4 and IL-5 induced TF-1 cell proliferaton**
**(IL-4:IL-5=3:1 )**

|  | Dupilumab(3:1) | Mepolizumab(3:1) | Sample B (3:1) |
|---|---|---|---|
| EC50 | 0.4481 | 0.001322 | 0.2429 |

FIG. 8

**Sample B  Neutralizing**

## IL-4 and IL-5 induced TF-1 cell proliferaton
## (IL-4:IL-5=3:1  )

| | Sample B (3:1) | Dupilumab+Mepolizumab(3:1) | hIgG(3:1) |
|---|---|---|---|
| EC50 | 0.2186 | 0.9652 | ~ 0.0009147 |

FIG. 9

**Sample B  Neutralizing**

## IL-4 and IL-5 induced TF-1 cell proliferaton
## (IL-4:IL-5=2:1 )

| | Dupilumab(2:1) | Mepolizumab(2:1) | Sample B (2:1) |
|---|---|---|---|
| EC50 | 0.4519 | ~ 2.936 | 0.2402 |

FIG. 10

**Sample B Neutralizing**
**IL-4 and IL-5 induced TF-1 cell proliferaton**
**(IL-4:IL-5=2:1 )**

|  | Sample B (2:1) | Dupilumab+Mepolizumab(2:1) | hIgG(2:1) |
|---|---|---|---|
| EC50 | 0.2127 | 1.279 | ~ |

FIG. 11

**Sample B Neutralizing**
**IL-4 and IL-5 induced TF-1 cell proliferaton**
**(IL-4:IL-5=1:1 )**

|  | Dupilumab(1:1) | Mepolizumab(1:1) | Sample B (1:1) |
|---|---|---|---|
| EC50 | 0.5701 | ~ 14.89 | 0.3692 |

FIG. 12

**Sample B Neutralizing**
**IL-4 and IL-5 induced TF-1 cell proliferaton**
**(IL-4:IL-5=1:1 )**

| | Sample B (1:1) | Dupilumab+Mepolizumab(1:1) | hIgG(1:1) |
|---|---|---|---|
| EC50 | 0.3709 | 1.733 | ~ 14.70 |

FIG. 13

FIG. 14

| | Dupilumab(1:2) | Mepolizumab(1:2) | Sample B (1:2) |
|---|---|---|---|
| EC50 | 0.8986 | ~ 0.001038 | 0.5947 |

| | Sample B (1:2) | Dupilumab+Mepolizumab(1:2) | hIgG(1:2) |
|---|---|---|---|
| EC50 | 0.7358 | 1.960 | 0.002641 |

FIG.15

**Sample B Neutralizing**
**IL-4 and IL-5 induced TF-1 cell proliferaton**
**(IL-4:IL-5=1:3 )**

| | Dupilumab(1:3) | Mepolizumab(1:3) | Sample B (1:3) |
|---|---|---|---|
| EC50 | 0.3429 | 0.001468 | 0.1523 |

FIG.16

Sample B **Neutralizing**
**IL-4 and IL-5 induced TF-1 cell proliferaton**
**(IL-4:IL-5=1:3 )**

| | Sample B (1:3) | Dupilumab+Mepolizumab(1:3) | hIgG(1:3) |
|---|---|---|---|
| EC50 | 0.1077 | 0.8805 | 0.001926 |

FIG. 17

Sample B **Neutralizing**
**IL-4 and IL-5 induced TF-1 cell proliferaton**
**(IL-4:IL-5=1:4 )**

| | Dupilumab(1:4) | Mepolizumab(1:4) | Sample B (1:4) |
|---|---|---|---|
| EC50 | 0.1562 | ~ 1.105 | 0.07883 |

FIG.18

FIG. 19

| | Sample B (1:4) | Dupilumab+Mepolizumab(1:4) | hIgG(1:4) |
|---|---|---|---|
| EC50 | 0.04987 | 0.3949 | ~ 0.000 |

## INTERNATIONAL SEARCH REPORT

| International application No. |
|---|
| **PCT/CN2023/088385** |

**A. CLASSIFICATION OF SUBJECT MATTER**

C07K16/46(2006.01)i; C07K16/28(2006.01)i; C12N15/13(2006.01)i; A61K39/395(2006.01)i; A61P11/06(2006.01)i

According to International Patent Classification (IPC) or to both national classification and IPC

**B. FIELDS SEARCHED**

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

TWMED; TWTXT; USTXT; DWPI; ENTXTC; KRTXT; CNTXT; WPABSC; JPTXT; CNABS; EPTXT; CNMED; WPABS; ENTXT; WOTXT; CJFD; CATXT; VEN, pubmed, genbank, elsevier science, 中国专利生物序列检索系统, China Patent Biological Sequence Search System: 单域, 抗体, single domain, antibody, IL-4, IL-5, 白介素-4, 白介素-5, 制剂, 稳定剂, 缓冲剂, 张度剂, 表面活性剂, formulation, buffer, tension agent, surfactant, 醋酸, 乙酸, acetic acid, NaCl, 氯化钠, 甘氨酸, glycine, 精氨酸, arginine, 甲硫氨酸, methionine, SEQ ID NO. 1-10.

**C. DOCUMENTS CONSIDERED TO BE RELEVANT**

| Category* | Citation of document, with indication, where appropriate, of the relevant passages | Relevant to claim No. |
|---|---|---|
| Y | CN 112041342 A (ARGENX BVBA) 04 December 2020 (2020-12-04)<br>see abstract, claims 36-59, embodiments 4-8, and figures 13-23 | 1-32, 34-36 |
| Y | CN 106661111 A (GLAXOSMITHKLINE INTELLECTUAL PROPERTY MANAGEMENT LIMITED) 10 May 2017 (2017-05-10)<br>see abstract, claims 1-24, and description, paragraphs 6, 7 and 72 | 1-32, 34-36 |
| Y | CN 111690066 A (NANJING REGENECORE BIOTECH CO., LTD.) 22 September 2020 (2020-09-22)<br>see abstract, claim 5, SEQ ID NO. 13 | 3, 8-10, 28-31, 34-36 |
| Y | CN 114075282 A (NANJING REGENECORE BIOTECH CO., LTD.) 22 February 2022 (2022-02-22)<br>see abstract, claims 1 and 2, description, paragraph 33, embodiment 1, and table 2 | 3, 8-10, 28-31, 34-36 |

☑ Further documents are listed in the continuation of Box C.

☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 August 2023** | **17 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/088385**

### C. DOCUMENTS CONSIDERED TO BE RELEVANT

| Category* | Citation of document, with indication, where appropriate, of the relevant passages | Relevant to claim No. |
|---|---|---|
| Y | US 2016257744 A1 (GENENTECH INC.) 08 September 2016 (2016-09-08) see abstract, claims 38, 39, 47-49 and 52-65, description, paragraph 124, embodiments 1-3, and figures 1-8 | 1-32, 34-36 |
| Y | WO 2022055299 A1 (AJOU UNIVERSITY INDUSTRY-ACADEMIC COOPERATION FOUNDATION) 17 March 2022 (2022-03-17) see abstract, and embodiments 12-33 | 1-32, 34-36 |
| Y | Marie Godar MSc. "A Bispecific Antibody Strategy to Target Multiple Type 2 Cytokines in Asthma" *Journal of Allergy and Clinical Immunology,* Vol. 142, No. (4), 31 October 2018 (2018-10-31), pages 1185-1193, see abstract, page 1187, left-hand column, paragraph 2 from the bottom to right-hand column, paragraph 1, and page 1188, paragraph 3 from the bottom to page 1191, paragraph 3 | 1-32, 34-36 |

Form PCT/ISA/210 (second sheet) (July 2022)

**INTERNATIONAL SEARCH REPORT**

International application No.

**PCT/CN2023/088385**

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

 a.  ☑ forming part of the international application as filed.

 b.  ☐ furnished subsequent to the international filing date for the purposes of international search (Rule 13*ter*.1(a)),

 ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2023/088385** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **33**
because they relate to subject matter not required to be searched by this Authority, namely:

The subject matter of claim 33 is a method for treating diseases, and specifically relates to treating diseases such as asthma by using the stable antibody preparation in the present application, which is a method for treatment of a living human or animal body by therapy, and falls within the cases set out in PCT Rule 39.1(iv) for which a search is not required.

2. ☑  Claims Nos.: **37, 38**
because they relate to parts of the international application that do not comply with the prescribed requirements to such an extent that no meaningful international search can be carried out, specifically:

Claim 37 sets forth the use of glycine in preparation of the stable antibody preparation of any one of claims 1-25, characterized in that: the antibody molecule still stably exists when the concentration thereof in the antibody preparation reaches 100 mg/ml.

The description of the present application provides six preparation formulas for verifying the stability of the preparation, wherein D1-D5 contain glycine; D5 is discarded due to significant stratification during solution replacement of an ultrafiltration centrifuge tube; formula D6 without glycine is not significantly different from other glycine-containing formulas D1-D4 in testing of pH, protein concentration, osmotic pressure, viscosity, purity, etc.; and finally, formula D4 containing glycine with the same concentration as that of D5 is the most preferred solution of all the formulas. In the present application, a single-factor experiment is not carried out on the influence of glycine on the stability of the preparation. Although glycine is a well-known component in the art that can be used for stabilizing an antibody preparation, on the basis of what is recited in the present application, it cannot be concluded that the technical effect of the antibody molecule of the preparation still stably existing when the concentration of the antibody molecule in the antibody preparation reaches 100mg/ml is brought about by glycine. Therefore, claim 37 is not supported by the description, and thus does not comply with PCT Article 6.

Claim 38 sets forth the use of the antibody preparation of any one of claims 1-25 in improving the stability of a high-concentration antibody in a liquid preparation. An experimental result of the description of the present application can merely indicate that the antibody of the present application can maintain certain stability in the partial preparation formulas, and the stability caused by the different formulas is only not completely the same but not significantly different. In the present application, whether the stability of the preparation is improved compared to other common or well-known formulas is not verified, and therefore there is no evidence that the preparation formulas improve the stability of the high-concentration antibody in the liquid preparation. Therefore, claim 38 is not supported by the description, and thus does not comply with PCT Article 6.

3. ☐  Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
| --- |
| **PCT/CN2023/088385** |

| Patent document cited in search report | | | Publication date (day/month/year) | Patent family member(s) | | | Publication date (day/month/year) |
| --- | --- | --- | --- | --- | --- | --- | --- |
| CN | 112041342 | A | 04 December 2020 | JP | 2021513974 | A | 03 June 2021 |
| | | | | US | 2020399382 | A1 | 24 December 2020 |
| | | | | GB | 201802487 | D0 | 04 April 2018 |
| | | | | WO | 2019158728 | A1 | 22 August 2019 |
| | | | | AU | 2019221627 | A1 | 06 August 2020 |
| | | | | IL | 276344 | A | 30 September 2020 |
| | | | | EP | 3752529 | A1 | 23 December 2020 |
| | | | | CA | 3088734 | A1 | 22 August 2019 |
| CN | 106661111 | A | 10 May 2017 | CA | 2949212 | A1 | 19 November 2015 |
| | | | | MX | 2016015006 | A | 28 September 2017 |
| | | | | EP | 3719038 | A1 | 07 October 2020 |
| | | | | AU | 2015260758 | A1 | 24 November 2016 |
| | | | | AU | 2015260758 | B2 | 02 November 2017 |
| | | | | SG | 11201609622 | PA | 29 December 2016 |
| | | | | BR | 112016026811 | A2 | 12 December 2017 |
| | | | | US | 2020222535 | A1 | 16 July 2020 |
| | | | | US | 11179463 | B2 | 23 November 2021 |
| | | | | ES | 2927990 | T3 | 14 November 2022 |
| | | | | MY | 185802 | A | 09 June 2021 |
| | | | | US | 2017095555 | A1 | 06 April 2017 |
| | | | | US | 10556009 | B2 | 11 February 2020 |
| | | | | WO | 2015173782 | A1 | 19 November 2015 |
| | | | | DK | 3143047 | T3 | 17 October 2022 |
| | | | | JP | 2020114819 | A | 30 July 2020 |
| | | | | JP | 7277681 | B2 | 19 May 2023 |
| | | | | US | 2018289804 | A1 | 11 October 2018 |
| | | | | US | 2022175922 | A1 | 09 June 2022 |
| | | | | JP | 2017515909 | A | 15 June 2017 |
| | | | | JP | 6707528 | B2 | 10 June 2020 |
| | | | | EP | 3143047 | A1 | 22 March 2017 |
| | | | | EP | 3143047 | B1 | 20 July 2022 |
| | | | | EP | 3143047 | B8 | 07 September 2022 |
| | | | | RU | 2016149151 | A | 20 June 2018 |
| | | | | RU | 2016149151 | A3 | 12 March 2019 |
| | | | | RU | 2743681 | C2 | 24 February 2021 |
| | | | | KR | 20170005099 | A | 11 January 2017 |
| | | | | IL | 248802 | A0 | 31 January 2017 |
| | | | | IL | 248802 | B | 27 February 2020 |
| | | | | KR | 20220148329 | A | 04 November 2022 |
| | | | | KR | 102501602 | B1 | 17 February 2023 |
| | | | | EP | 3715371 | A1 | 30 September 2020 |
| | | | | TW | 201601756 | A | 16 January 2016 |
| | | | | TWI | 694836 | B | 01 June 2020 |
| CN | 111690066 | A | 22 September 2020 | None | | | |
| CN | 114075282 | A | 22 February 2022 | None | | | |
| US | 2016257744 | A1 | 08 September 2016 | MX | 2021003549 | A | 27 May 2021 |
| | | | | CA | 3204788 | A1 | 25 June 2015 |
| | | | | EP | 3083682 | A1 | 26 October 2016 |
| | | | | KR | 20160099086 | A | 19 August 2016 |
| | | | | JP | 2020162609 | A | 08 October 2020 |

Form PCT/ISA/210 (patent family annex) (July 2022)

## INTERNATIONAL SEARCH REPORT
### Information on patent family members

International application No.

**PCT/CN2023/088385**

| Patent document cited in search report | | | Publication date (day/month/year) | Patent family member(s) | | | Publication date (day/month/year) |
|---|---|---|---|---|---|---|---|
| | | | | RU | 2020129339 | A | 02 October 2020 |
| | | | | CA | 2931113 | A1 | 25 June 2015 |
| | | | | CA | 2931113 | C | 11 July 2023 |
| | | | | WO | 2015095539 | A1 | 25 June 2015 |
| | | | | US | 2020255512 | A1 | 13 August 2020 |
| | | | | MX | 2016006529 | A | 03 August 2016 |
| | | | | RU | 2016129247 | A | 25 January 2018 |
| | | | | RU | 2732032 | C2 | 10 September 2020 |
| | | | | US | 10683348 | B2 | 16 June 2020 |
| | | | | JP | 2017501711 | A | 19 January 2017 |
| | | | | BR | 112016011027 | A2 | 05 December 2017 |
| WO | 2022055299 | A1 | 17 March 2022 | KR | 20220035655 | A | 22 March 2022 |
| | | | | EP | 4212550 | A1 | 19 July 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010576200

- CN 202010843501

**Non-patent literature cited in the description**

- **WANG et al.** *J. of parenteral Science & Technology*, 1988, vol. 42, S4-S26
- **CLELAND et al.** *Critical Reviews in Therapeutic Drug Carrier Systems*, 1993, vol. 10 (4), 307-377
- Peptide and Protein Drug Delivery. Marcel Dekker, Inc., 1991, 247-301
- **JONES, A.** *Adv. Drug Delivery Rev.*, 1993, vol. 10, 29-90
- Measurement of pH Value. Pharmacopoeia of the People's Republic of China. 2015
- Molar concentration assay for osmotic pressure. Pharmacopoeia of the People's Republic of China. 2015